(19) Europäisches Patentamt
European Patent Office
Office européen des brevets

(11) **EP 4 183 873 A1**

(12) **EUROPEAN PATENT APPLICATION**
published in accordance with Art. 153(4) EPC

(43) Date of publication:
**24.05.2023 Bulletin 2023/21**

(21) Application number: **20945461.0**

(22) Date of filing: **17.07.2020**

(51) International Patent Classification (IPC):
**C12N 5/10** $^{(2006.01)}$    **C12N 15/10** $^{(2006.01)}$

(52) Cooperative Patent Classification (CPC):
**C12N 5/10; C12N 15/10**

(86) International application number:
**PCT/JP2020/027766**

(87) International publication number:
**WO 2022/014028 (20.01.2022 Gazette 2022/03)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Tohoku University
Sendai-shi, Miyagi 980-8577 (JP)**

(72) Inventors:
• **OKAE Hiroaki
Sendai-shi, Miyagi 980-8577 (JP)**

• **ARIMA Takahiro
Sendai-shi, Miyagi 980-8577 (JP)**

(74) Representative: **Cabinet Becker et Associés
25, rue Louis le Grand
75002 Paris (FR)**

Remarks:
The complete document including Reference Table(s) and the Sequence Listing(s) can be downloaded from the EPO website

(54) **TROPHOBLAST STEM CELL-LIKE CELLS CAPABLE OF DIFFERENTIATING INTO PLACENTA-CONSTITUTING CELLS, AND METHOD FOR PRODUCING SAME**

(57)    A trophoblast stem cell-like cell having potential to differentiate into a placenta-constituting cell which is induced from a trophoblast cell derived from a placenta of or after the second trimester of pregnancy and which contains a SALL4 gene functionally linked to a first exogenous inducible promoter. Also, a method for producing a trophoblast stem cell-like cell having potential to differentiate into a placenta-constituting cell.

EP 4 183 873 A1

**Description**

Technical Field

The present invention relates to trophoblast stem cell-like cells having potential to differentiate into placenta constituting cells and a production method thereof.

Background Art

Trophoblast cells are a major component of placental tissue. Trophoblast stem cells (TS cells) are believed to be very useful for elucidating the mechanism of regulation of differentiation in trophoblast cells. Therefore, establishment of a TS cell line which can be passaged while maintaining the undifferentiated state is desired.
The present inventors have developed a method for collecting CD49f-positive cells from cells suspension obtained from placental tissue in the first trimester of pregnancy and inducing differentiation into cells having similar features to those of TS cells (TS-like cells) (PTL 1 and NPL 1).

Citation List

Patent Literature

PTL 1: Patent No. 6400832

Non Patent Literature

NPL 1: Okae H, et al., Derivation of Human Trophoblast Stem Cells. Cell Stem Cell. 2018 Jan; 22: 50-63.

Summary of Invention

Technical Problem

The method described in PTL 1 and NPL 1 can induce the differentiation from cells obtained from placental tissue of the first trimester of pregnancy (cytotrophoblast cells: CT cells) into TS-like cells but cannot induce the differentiation from CT cells derived from a placenta of or after the second trimester of pregnancy into TS-like cells. Diseases such as pregnancy induced hypertension, incomplete placental development, underweight and trisomies become evident during and after the second trimester of pregnancy. Accordingly, when the differentiation from CT cells derived from a placenta of or after the second trimester of pregnancy into TS-like cells can be induced, it is useful for researching the diseases. Moreover, when a placenta after birth can be used, TS-like cells can be more easily produced.
Therefore, an object of the invention is to provide a method for producing TS-like cells from trophoblast cells derived from a placenta of or after the second trimester of pregnancy and TS-like cells produced by the production method.

Solution to Problem

The invention includes the following aspects.

[1] A trophoblast stem cell-like cell having potential to differentiate into a placenta-constituting cell which is induced from a trophoblast cell derived from a placenta of or after the second trimester of pregnancy and which contains a SALL4 gene functionally linked to a first exogenous inducible promoter.
[2] The trophoblast stem cell-like cell described in [1], wherein the SALL4 gene is an exogenous SALL4 gene functionally linked to the first exogenous inducible promoter or an endogenous SALL4 gene functionally linked to the first exogenous inducible promoter.
[3] The trophoblast stem cell-like cell described in [1] or [2] which has at least one feature selected from the group consisting of (a) and (b) below: (a) the activity of p53 is suppressed compared to that in the trophoblast cell; and (b) the expression of a MYC gene is promoted compared to that in the trophoblast cell.
[4] The trophoblast stem cell-like cell described in any one of [1] to [3] which further contains an exogenous gene functionally linked to a second exogenous inducible promoter, wherein the exogenous gene is at least one selected from the group consisting of an exogenous p53 dominant negative gene and an exogenous MYC gene.
[5] The trophoblast stem cell-like cell described in [4], wherein the exogenous gene is the exogenous p53 dominant negative gene.

[6] A method for producing a trophoblast stem cell-like cell having potential to differentiate into a placenta-constituting cell, including (i) a step of preparing a trophoblast cell derived from a placenta of or after the second trimester of pregnancy, (ii) a step of inducing the expression of a SALL4 gene in the trophoblast cell and (iii) a step of conducting at least one selected from the group consisting of (A) and (B) below: (A) suppressing the activity of p53 of the trophoblast cell; and (B) inducing the expression of a MYC gene in the trophoblast cell.

[7] The method for producing a trophoblast stem cell-like cell described in [6],

wherein the step in (ii) includes introducing a polynucleotide containing an exogenous SALL4 gene functionally linked to a first exogenous inducible promoter into the trophoblast cell or introducing a polynucleotide containing the first exogenous inducible promoter into the trophoblast cell at the upstream of an endogenous SALL4 gene in such a manner that the endogenous SALL4 gene is functionally linked to the first exogenous inducible promoter and

inducing the expression of the exogenous SALL4 gene or the endogenous SALL4 gene with a first inducing factor which induces the transcriptional activity of the first exogenous inducible promoter.

[8] The method for producing a trophoblast stem cell-like cell described in [7], wherein the (A) in the step in (iii) includes introducing a polynucleotide containing an exogenous p53 dominant negative gene functionally linked to a second exogenous inducible promoter into the trophoblast cell and inducing the expression of the exogenous p53 dominant negative gene with a second inducing factor which induces the transcriptional activity of the second exogenous inducible promoter, and the (B) in the step in (iii) includes introducing an exogenous MYC gene functionally linked to the second exogenous inducible promoter into the trophoblast cell and inducing the expression of the exogenous MYC gene with a second inducing factor which induces the transcriptional activity of the second exogenous inducible promoter.

[9] The method for producing a trophoblast stem cell-like cell described in any one of [6] to [8], wherein the step in (iii) includes the (A).

Advantageous Effects of Invention

According to the invention, a method for producing TS-like cells from trophoblast cells derived from a placenta of or after the second trimester of pregnancy and TS-like cells produced by the production method are provided.

Brief Description of Drawings

[Fig. 1] The structure of CS-CA-MCS plasmid used for the construction of Dox-inducible gene introduction lentivectors is shown.

[Fig. 2] The outline of the time schedule of a method for inducing TS-like cells from CT cells derived from a placenta of or after the second trimester of pregnancy (second/third-trimester CT cells) is shown.

[Fig. 3] Optical microscope images of cells obtained by introducing a Doxycycline (Dox)-inducible SALL4 gene and a Dox-inducible p53 dominant negative (p53DN) gene into second/third-trimester CT cells and culturing the cells are shown. The cells of Day 12 and Day 17 were cultured in a Dox-containing medium, and the cells of Day 40 were cultured in a Dox-free medium. The shape of TS-like cells was maintained also at Day 40.

[Fig. 4] Optical microscope images of cells obtained by introducing a Doxycycline (Dox)-inducible SALL4 gene and a Dox-inducible MYC gene into second/third-trimester CT cells and culturing the cells are shown. The cells of Day 6 and Day 15 were cultured in a Dox-containing medium, and the cells of Day 29 were cultured in a Dox-free medium. The shape of TS-like cells was not maintained at Day 29.

[Fig. 5] The results of main component analysis of gene expression in TS-like cells induced from second/third-trimester CT cells (second/third-trimester TS-like cells), TS-like cells induced from first-trimester placenta-derived CT cells (first-trimester TS-like cells), TS cells, second/third-trimester CT cells and first-trimester CT cells are shown.

[Fig. 6] The results of expression analysis of TS cell markers (positive markers ELF5 and ZNF750 and negative marker CDX2) in first-trimester TS-like cells and second/third-trimester TS-like cells are shown.

[Fig. 7A] Induced differentiation from TS cells into extravillous cytotrophoblast (EVT) cells and into syncytiotrophoblast (ST) cells are shown.

[Fig. 7B] An optical microscope image of induced differentiation into EVTs from TS-like cells induced by introducing SALL4 gene and p53DN gene into second/third-trimester CT cells is shown.

[Fig. 7C] An optical microscope image of induced differentiation into STs from TS-like cells induced by introducing SALL4 gene and p53DN gene into second/third-trimester CT cells is shown.

[Fig. 8] The results of expression analysis of CCR7 in first-trimester CT cells and second/third-trimester CT cells are shown.

[Fig. 9] The results of expression analysis of TS cell markers (positive markers ELF5 and ZNF750 and negative marker CDX2) in disease CT cell-derived TS-like cells and healthy CT cell-derived TS-like cells are shown.

Description of Embodiments

[Definition]

The terms "polynucleotide" and "nucleic acid" are used interchangeably and refer to a nucleotide polymer in which nucleotides are bonded through phosphodiester bonds. The "polynucleotide" and the "nucleic acid" may be DNA or RNA and may be composed of a combination of DNA and RNA. The "polynucleotide" and the "nucleic acid" may be a polymer of natural nucleotides, a polymer of natural nucleotides and unnatural nucleotides (analogs of natural nucleotides, nucleotides in which at least one of a base moiety, a sugar moiety and a phosphate moiety is modified (for example, phosphorothioate structure) and the like) or a polymer of unnatural nucleotides.

The base sequence of the "polynucleotide" or the "nucleic acid" is described with the generally accepted single-letter code unless otherwise specified. Unless otherwise specified, the base sequence is described from the 5' end to the 3' end.

The nucleotide residues constituting the "polynucleotide" or the "nucleic acid" are sometimes simply described with adenine, thymine, cytosine, guanine, uracil and the like or with the single-letter code thereof.

The term "gene" refers to a polynucleotide containing at least one open reading frame encoding a specific protein. The gene can contain both an exon and an intron.

The terms "polypeptide", "peptide" and "protein" are used interchangeably and refer to a polymer of amino acids bonded through amide bonds. The "polypeptide", the "peptide" or the "protein" may be a polymer of natural amino acids, a polymer of natural amino acids and unnatural amino acids (chemical analogs, modified derivatives and the like of natural amino acids) or a polymer of unnatural amino acids. Unless otherwise specified, the amino acid sequence is described from the N-terminal to the C-terminal.

The term "functionally linked" used for a polynucleotide means that a first base sequence is located sufficiently close to a second base sequence and that the first base sequence can affect the second base sequence or a region under the regulation of the second base sequence. For example, that a polynucleotide is functionally linked to a promoter means that the polynucleotide is linked in such a manner that the polynucleotide is expressed under the regulation of the promoter.

The term "expressible state" refers to the state in which a polynucleotide can be transcribed in the cells into which the polynucleotide has been introduced.

The term "expression vector" refers to a vector which contains a target polynucleotide and which has a system for bringing the target polynucleotide into an expressible state in the cells into which the vector has been introduced.

The term "endogenous" means that cells naturally have. An endogenous polynucleotide is a polynucleotide which cells naturally contain in the genome (the nuclear genome or the organelle genome). An endogenous polypeptide is a polypeptide which is produced through transcription and translation of an endogenous polynucleotide.

The term "exogenous" refers to a factor given to cells from the outside. An exogenous polynucleotide refers to a polynucleotide introduced into cells from the outside. An exogenous polypeptide is a polypeptide which is produced through transcription and translation from an exogenous polynucleotide or a polypeptide supplied to cells from the outside. An exogenous factor may be an exogenous chemical substance, an exogenous physiological condition (for example, pH, temperature, radiation, osmotic pressure or saline gradient) or the like.

The term "inducible promoter" refers to a promoter in which the transcriptional activity is initiated or enhanced in the presence of an inducing factor compared to the case without the presence of the inducing factor. That is, in the presence of the inducing factor, the transcription of a gene functionally linked to the inducible promoter is initiated or enhanced.

The term "inducing factor" refers to a factor which induces the transcriptional activity of an inducible promoter. The inducing factor is preferably an exogenous stimulus. Examples of the exogenous stimulus include an exogenous chemical substance, an exogenous physiological condition (for example, pH, temperature, light, radiation, osmotic pressure or saline gradient) and the like.

The term "dominant negative" refers to a polypeptide which functions dominantly over the normal polypeptide and which has an action of inhibiting the action of the normal polypeptide. The dominant negative may be an inactive form mutant of the normal polypeptide. The inactive form mutant which functions as dominant negative can be a mutant which maintains the binding activity to the ligand of the normal polypeptide and which does not exhibit the function that is exhibited when the normal polypeptide binds to the ligand.

[Trophoblast Stem Cell-Like Cells (TS-Like Cells)]

In an embodiment, a trophoblast stem cell-like cell having potential to differentiate into a placenta-constituting cell which is induced from a trophoblast cell derived from a placenta of or after the second trimester of pregnancy and which contains a SALL4 gene functionally linked to a first exogenous inducible promoter is provided.

The cells of the embodiment are TS-like cells having similar features to those of TS cells. Like TS cells, the TS-like cells have potential to differentiate into placenta constituting cells. Examples of the placenta-constituting cells include extravillous cytotrophoblast (EVT) cells and syncytiotrophoblast (ST) cells.

Whether or not cells have potential to differentiate into EVT can be determined by culturing the cells under conditions which are used as conditions for inducing differentiation into an EVT and by observing whether the cells differentiate into an EVT. The conditions for inducing differentiation into an EVT are, for example, the conditions described in the Examples described below. Specifically, differentiation into an EVT can be induced as follows.

Cells are inoculated on a Collagen IV (Col IV)-coated plate containing an EVT medium (see the Examples), and the cells are cultured after adding Matrigel at 2% of the medium volume. On the third day after the inoculation, the medium is replaced with an NRG1-free EVT medium, and the cells are further cultured after adding Matrigel at 0.5% of the medium volume. On the sixth day after the inoculation, the medium is replaced with an NRG1- and KSR-free EVT medium, and the cells are further cultured for six to eight days after adding Matrigel at 0.5% of the medium volume.

Whether or not cells cultured under the conditions for inducing differentiation into an EVT has differentiated into an EVT can be determined through morphological observation under a microscope and also with an increase in the expression of HLA-G. HLA-G is a marker of EVTs, and the expression thereof is up-regulated by differentiation from TS cells into an EVT. Accordingly, when the expression level of HLA-G is increased in cells after culturing under the conditions compared to that in the cells before culturing under the conditions, it can be determined that the cells have differentiated into an EVT.

Examples of HLA-G (NCBI Gene ID:3135) include those registered with GenBank accession number NM_001363567.1 or NM_002127.5 and the like.

Whether or not cells have potential to differentiate into an ST can be determined by culturing the cells under conditions which are used as conditions for inducing differentiation into an ST and by observing whether the cells differentiate into an ST. The conditions for inducing differentiation into an ST are, for example, the conditions described in the Examples described below. Specifically, differentiation into an ST can be induced as follows.

Cells are inoculated on a Collagen IV (Col IV)-coated plate containing an ST medium (see the Examples), and the cells are cultured. On the third day after the inoculation, the medium is replaced with fresh ST medium, and the cells are further cultured for three days.

Whether or not cells cultured under the conditions for inducing differentiation into an ST has differentiated into an ST can be determined through morphological observation under a microscope and also with an increase in the expression of CGβ. CGβ (another name: CGB3 (chorionic gonadotropin subunit beta 3)) is a marker of STs, and the expression thereof is up-regulated by differentiation from TS cells into an ST. Accordingly, when the expression level of CGβ is increased in cells after culturing under the conditions compared to that in the cells before culturing under the conditions, it can be determined that the cells have differentiated into an ST.

Examples of human CGβ (NCBI Gene ID:1082) include one registered with GenBank accession number NM_000737.3 and the like.

The cells of the embodiment are TS-like cells having similar features to those of TS cells and expresses a TS cell marker. Examples of the TS cell marker include ELF5 (E74 like ETS transcription factor 5), ZNF750 (zinc finger protein 750) and the like. Examples of human ELF5 (NCBI Gene ID:2001) include one registered with GenBank accession number NM_001243080.2 and the like. Examples of human ZNF750 (NCBI Gene ID:79755) include one registered with GenBank accession number NM_024702.3 and the like.

The expression of CDX2 (caudal type homeobox 2) is low in the cells of the embodiment as in TS cells. Examples of human CDX2 (NCBI Gene ID:1045) include one registered with GenBank accession number NM_001265.6 and the like.

<Trophoblast Cell>

The cells of the embodiment are induced from trophoblast cells derived from a placenta of or after the second trimester of pregnancy. The "of or after the second trimester of pregnancy" means the gestational period at or after 1/3 of the gestational period and after birth. The "placenta of or after the second trimester of pregnancy" means a placenta at or after 1/3 of the gestational period and may be a placenta of the gestational period or a placenta after birth.

The placenta is not particularly limited as long as the placenta is of a placental mammal in which a placenta is formed during pregnancy. Examples of the placental mammal include primates (human, chimpanzee, rhesus macaque, marmoset and the like), rodents (mouse, rat, hamster, guinea pig and the like), carnivores (dog, cat, weasel and the like) and the like. The placental mammal is preferably a primate, more preferably a human.

When a human placenta is used, the "of or after the second trimester of pregnancy" refers to the gestational period in and after 12 weeks of gestation and after birth. The human placenta of or after the second trimester of pregnancy may be a placenta of the gestational period in or after 12 weeks of gestation and may be a placenta after birth. As the human placenta in or after 12 weeks of gestation, for example, a placenta expelled from the body due to induced abortion, spontaneous abortion or birth can be used.

The placenta may be a placenta of normal pregnancy (also called "normal placenta" below) or a placenta of abnormal pregnancy (also called "disease placenta" below). For example, the placenta may be the placenta of an individual which has developed a disease such as pregnancy induced hypertension, oligoamnios, hydramnios, fetal growth restriction, low birth weight birth, chromosomal abnormalities (trisomies and the like), spontaneous abortion and premature birth. TS-like cells induced from the placenta of an individual which have developed a disease can be used for researching the disease and developing a therapeutic agent and a therapeutic method for the disease.

The "trophoblast cells derived from a placenta of or after the second trimester of pregnancy" (also called "second/third-trimester trophoblast cells" below) means trophoblast cells isolated from a placenta of or after the second trimester of pregnancy. The trophoblast cells are preferably cytotrophoblast cells (CT cells). The CT cells are precursor cells in placental tissue. While CT cells derived from a placenta of the first trimester of pregnancy can be differentiated into TS-like cells by adjusting the medium components, CT cells derived from a placenta of or after the second trimester of pregnancy cannot be differentiated into TS-like cells even when the medium components are adjusted. When the method described below is used, however, TS-like cells can be induced from CT cells derived from a placenta of or after the second trimester of pregnancy. The TS-like cells of the embodiment are TS-like cells produced by the method described below.

The method for isolating trophoblast cells from a placenta of or after the second trimester of pregnancy is not particularly limited. A specific example thereof is, for example, the method described in the Examples. For example, cells suspension can be prepared from placental tissue of or after the second trimester of pregnancy appropriately using mechanical and/or enzymatic treatment. For example, by mincing placental tissue into small pieces, appropriately washing with physiological saline or the like, immersing in a cell-dispersing solution and another procedure, cells suspension may be prepared.

As the cell-dispersing solution, for example, those which are called a protease solution, cells adhesion detachment solution and the like may be used. Specific examples of the cell-dispersing solution include, for example, those commercially available with product names of Accumax, TrypLE and the like and the like. One kind of cell-dispersing solution may be used alone, or a mixture of two or more kinds may be used. After treatment with the cell-dispersing solution, cells may be further dispersed using a cell strainer or the like. The cell suspension prepared in the above manner may be subjected to treatment for removing erythrocytes. Examples of the method for removing erythrocytes include treatment with colloidal silica particles coated with polyvinylpyrrolidone (one commercially available as Percoll (registered trademark) or the like) and the like.

Next, CD49f antibody-positive cells are collected from the cell suspension. The method for collecting CD49f antibody-positive cells is not particularly limited, and a known method can be used. Examples thereof include a method using an anti-CD49f antibody and the like.

After the collection or before the collection of CD49f antibody-positive cells, treatment for removing CD45 antibody-positive cells may be conducted. The method for removing CD45 antibody-positive cells is not particularly limited, and a known method can be used. Examples thereof include a method using an anti-CD45 antibody and the like.

The trophoblast cells isolated in the above manner can be maintained in a medium containing a growth factor and a ROCK inhibitor.

The second/third-trimester trophoblast cells (preferably a CT cells) are characterized in that the expression of CCR7 is lower than that in trophoblast cells (preferably CT cells) derived from a placenta of the first trimester of pregnancy (before 1/3 of the gestational period). The expression of CCR7 is low regardless of whether the cells are a normal placenta-derived second/third-trimester trophoblast cells or a disease placenta-derived second/third-trimester trophoblast cells. More specifically, in the second/third-trimester trophoblast cells, the TPM (transcripts per million) of CCR7 is preferably 50 or less, 40 or less, 30 or less or 20 or less. On the other hand, in the trophoblast cells derived from a placenta of the first trimester of pregnancy (also called "first-trimester trophoblast cells" below), the TPM of CCR7 is preferably 100 or more, 150 or more, 160 or more, 170 or more or 180 or more. The TPM is a value which can be obtained using a next generation sequencer and is a value indicating the number of the target transcripts when there are one million transcripts in total in the sample. The TPM of a transcript t ($TPM_t$) can be determined by the following equation.

[Math. 1]

$$\mathrm{TPM}_t = T_t \frac{1}{\sum_t T_t} 10^6$$

In the above equation, $T_t$ is the read count of the transcript t per 1,000 bp and can be calculated by the following equation.

[Math. 2]

$$T_t = \frac{Y_t}{L_t} 10^3$$

In the above equation, Yt is the count of reads mapped to the transcript t, and $L_t$ is the length of the transcript t.

A CCR7 (C-C motif chemokine receptor 7) gene encodes the G protein-coupled receptor family. The sequence of a CCR7 gene is known and can be obtained from a known database such as GenBank. Examples of human CCR7 (NCBI Gene ID:1236) include one registered with GenBank accession number NM_001301714.2 and the like.

The expression of CCR7 is low in the second/third-trimester trophoblast cells as the starting material of the TS-like cells of the embodiment but is high in the TS-like cells of the embodiment.

<SALL4 Gene Functionally Linked to First Exogenous Inducible Promoter>

The TS-like cells of the embodiment are characterized by containing a SALL4 gene functionally linked to a first exogenous inducible promoter. Accordingly, the TS-like cells can be distinguished from TS cells in that a SALL4 gene functionally linked to a first exogenous inducible promoter is contained.

(First Exogenous Inducible Promoter)

The inducible promoter used as the first exogenous inducible promoter is not particularly limited, and a known inducible promoter can be used. The inducible promoter is preferably an inducible promoter in which the transcriptional activity is induced with an exogenous inducing factor. Examples of the first exogenous inducible promoter include a promoter in which the transcriptional activity is induced with an exogenous chemical substance, a promoter in which the transcriptional activity is induced with an exogenous physiological condition (for example, pH, temperature, light, radiation, osmotic pressure or saline gradient) and the like. Examples of the inducible promoter include a tetracycline-inducible promoter, a lac operon promoter, an IPTG-inducible promoter, a steroid-inducible promoter, a rapamycin-inducible promoter, a temperature-inducible promoter, a pH-inducible promoter, a salt-inducible promoter, a radiation-inducible promoter and the like, but the inducible promoter is not limited thereto.

The tetracycline-inducible promoter is an inducible promoter in which the transcriptional activity is induced in the presence of a tetracycline or an analog thereof. The tetracycline-inducible promoter preferably contains a minimal promoter functionally linked to a Tet operator (tetO) sequence. A transcription activator binds to the tetO sequence in the presence of a tetracycline or an analog thereof, and the transcriptional activity of the minimal promoter is induced. As a result, the transcription of a gene functionally linked to the minimal promoter is induced. The "tetracycline analog" is a compound which has a structural homology to a tetracycline and which can induce the transcriptional activity of the tetracycline-inducible promoter. The tetracycline analog is not particularly limited, but examples thereof include doxycycline (Dox), chlortetracycline, anhydrotetracycline and the like. The "minimal promoter" means a promoter having the minimum sequence as a promoter. The minimal promoter can be considered as a promoter containing all the elements which are necessary for appropriately initiating the transcription of the functionally linked gene. The minimal promoter can contain, for example, a transcription start site, an RNA polymerase binding site and the TATA box. Examples of the minimal promoter include a thymidine kinase promoter, a β-globulin promoter, a cytomegalovirus (CMV) promoter, a SV40 promoter and the like. When the inducible promoter is a tetracycline-inducible promoter, the TS-like cells preferably further contain a transcription activator (for example, reverse tetracycline-controlled transactivator: rtTA)-coding sequence which is functionally linked to an appropriate promoter.

As an expression system containing the tetracycline-inducible promoter, a commercial system may be used. For example, pTetOne vector (Takara) and the like can be used. Moreover, as an expression system containing another inducible promoter, a commercial system can be used.

(SALLL4 Gene)

The SALL4 (spalt like transcription factor 4) gene encodes SALL4 which is a transcription factor containing a zinc finger. The sequence of the SALL4 gene is known and can be obtained from a known database such as GenBank. Examples of human SALL4 (NCBI Gene ID:57167) include those registered with GenBank accession number NM_001318031.2 (SEQ ID NOs: 1 and 2) or NM_020436.5 (SEQ ID NOs: 3 and 4) and the like. The human SALL4 gene is not limited to the examples and may be a homolog (an ortholog or a paralog) thereof or a mutant thereof. The sequence information of SALL4 genes that other mammals have can also be obtained from a known database such as GenBank.

The SALL4 gene functionally linked to the first exogenous inducible promoter may be an endogenous SALL4 gene or an exogenous SALL4 gene.

When the SALL4 gene functionally linked to the first exogenous inducible promoter is endogenous, the first exogenous inducible promoter may be a promoter inserted at the upstream of the endogenous SALL4 gene using a genome editing technique or the like. As the genome editing technique, a known technique can be used, and examples thereof include CRISPR/Cas system, a zinc finger nuclease (ZFN), a transcription activator-like effector nuclease (TALEN) and the like.

When the first exogenous inducible promoter is a tetracycline-inducible promoter, a Tet operator (tetO) sequence (for example, a tetO repeat sequence-having tetracycline response element: TRE) may be introduced at the upstream of the promoter of the endogenous SALL4 gene in such a manner that the endogenous SALL4 gene promoter is functionally linked.

When the SALL4 gene functionally linked to the first exogenous inducible promoter is exogenous, the exogenous SALL4 gene functionally linked to the first exogenous inducible promoter (also called "exogenous SALL4 gene expression cassette" below) may be one introduced from the outside using a known gene transfer technique. The exogenous SALL4 expression cassette may be in the genome (for example, the nuclear genome) or may be outside the genome as a plasmid or the like.

The SALL4 gene functionally linked to the first exogenous inducible promoter is preferably an exogenous SALL4 gene. The exogenous SALL4 gene functionally linked to the first exogenous inducible promoter is preferably on the nuclear genome.

When the SALL4 gene is exogenous, the organism from which the exogenous SALL4 gene is derived is preferably the same as the organism from which the second/third-trimester trophoblast cells are derived. For example, when a human second/third-trimester trophoblast cells are used, a human SALL4 gene is preferable.

The exogenous SALL4 gene is not limited to a wild-type gene and may contain mutation (any of deletion, substitution, insertion and addition) as long as the exogenous SALL4 gene has ability of inducing into TS-like cells. The "ability of inducing into TS-like cells" means a function which induces differentiation of second/third-trimester trophoblast cells into TS-like cells when the expression of the gene is induced.

Examples of the exogenous SALL4 gene include (A) to (G) below.

(A) A wild-type SALL4 gene (for example, a polynucleotide having the nucleotide sequence of SEQ ID NO: 1 or 3) .
(B) A polynucleotide having a nucleotide sequence encoding a wild-type SALL4 protein (for example, a protein having the amino acid sequence of SEQ ID NO: 2 or 4).
(C) A polynucleotide which encodes a protein having an amino acid sequence of a wild-type SALL4 protein (for example, the amino acid sequence of SEQ ID NO: 2 or 4) having mutation of one amino acid or a plurality of amino acids and which has an ability of inducing into TS-like cells.
(D) A polynucleotide which encodes a protein having an amino acid sequence having a sequence identity of 70% or more with the amino acid sequence of a wild-type SALL4 protein (for example, the amino acid sequence of SEQ ID NO: 2 or 4) and which has an ability of inducing into TS-like cells.
(E) A polynucleotide which has a nucleotide sequence of a wild-type SALL4 gene (for example, a polynucleotide having the nucleotide sequence of SEQ ID NO: 1 or 3) having mutation of one nucleotide or a plurality of nucleotides and which has an ability of inducing into TS-like cells.
(F) A polynucleotide which has a nucleotide sequence having a sequence identity of 70% or more with a wild-type SALL4 gene (for example, a polynucleotide having the nucleotide sequence of SEQ ID NO: 1 or 3) and which has an ability of inducing into TS-like cells.
(G) A polynucleotide which hybridizes to a wild-type SALL4 gene (for example, a polynucleotide having the nucleotide sequence of SEQ ID NO: 1 or 3) under stringent conditions and which has an ability of inducing into TS-like cells.

In (C) and (E) above, the "mutation" may be any of deletion, substitution, addition and insertion or a combination thereof.

In (C) above, the "plurality" is not particularly limited but is, for example, 2 to 60 residues, 2 to 50 residues, 2 to 40 residues, 2 to 30 residues, 2 to 20 residues, 2 to 15 residues, 2 to 10 residues, 2 to 90 residues, 2 to 8 residues, 2 to 7 residues, 2 to 6 residues, 2 to 5 residues, 2 to 4 residues, 2 or 3 residues or 2 residues.

In (E) above, the "plurality" is not particularly limited but is, for example, 2 to 100 residues, 2 to 90 residues, 2 to 80 residues, 2 to 70 residues, 2 to 60 residues, 2 to 50 residues, 2 to 40 residues, 2 to 30 residues, 2 to 20 residues, 2 to 15 residues, 2 to 10 residues, 2 to 9 residues, 2 to 8 residues, 2 to 7 residues, 2 to 6 residues, 2 to 5 residues, 2 to 4 residues, 2 or 3 residues or 2 residues.

In (D) and (F) above, the sequence identity is not particularly limited as long as the sequence identity is 70% or more, but the sequence identity is 80% or more, 85% or more, 90% or more, 95% or more, 96% or more, 97% or more, 98% or more or 99% or more. The sequence identity of amino acid sequences or nucleotide sequences is determined as the ratio of matching amino acids or nucleotides to the whole amino acid sequence or the whole nucleotide sequence excluding the gaps in the alignment obtained by aligning two amino acid sequences or nucleotide sequences while inserting gaps to the sites corresponding to insertions and deletions so that the corresponding amino acids or nucleotides match the most. The sequence identity of amino acid sequences or nucleotide sequences can be determined using homology search software known in the technical field. For example, the sequence identity value of amino acid sequences or nucleotide sequences can be obtained through calculation based on an alignment obtained by known homology search software, BLASTP.

In (G) above, the "stringent conditions" are, for example, the conditions described in Molecular Cloning-A LABORATORY MANUAL THIRD EDITION (Sambrook et al., Cold Spring Harbor Laboratory Press). An example thereof is the conditions for hybridization through incubation at 42 to 70°C for several hours to overnight in a hybridization buffer containing 6×SSC (composition of 20×SSC: 3M sodium chloride and 0.3M citric acid solution, pH7.0), 5×Denhardt's solution (composition of 100×Denhardt's solution: 2 mass% bovine serum albumin, 2 mass% Ficoll and 2 mass% polyvinylpyrrolidone), 0.5 mass% SDS, 0.1 mg/mL salmon sperm DNA and 50% formamide. In this regard, the wash buffer used for washing after the incubation is preferably 0.1 mass% SDS-containing 1×SSC solution, more preferably 0.1 mass% SDS-containing 0.1×SSC solution.

In (B) to (E) above, as the degenerate codons, codons with high usage in the cells of the placental mammal used are preferably used. For example, when used for human second/third-trimester trophoblast cells, codons with high usage in human cells are preferably used. That is, the codons are preferably optimized for human codons.

The SALL4 gene functionally linked to the first exogenous inducible promoter is preferably an exogenous SALL4 gene.

<Optional Constitution>

The TS-like cells of the embodiment may have at least one feature selected from the group consisting of (a) and (b) below in addition to containing the SALL4 gene functionally linked to the first exogenous inducible promoter.

(a) The activity of p53 is suppressed compared to that in the trophoblast cells.
(b) The expression of a MYC gene is promoted compared to that in the trophoblast cells.

(Feature (a): Suppression of p53 Activity)

In the TS-like cells of the embodiment, the activity of p53 may be suppressed compared to that in the second/third-trimester trophoblast cells. p53 is a tumor suppressor protein and has a function of regulating suppression of the cell growth cycle such as DNA repair, cell growth arrest and apoptosis. The sequence of a p53 gene is known and can be obtained from a known database such as GenBank. Examples of human p53 (NCBI Gene ID:7157) include one registered with AB082923.1 (SEQ ID NOs: 5 and 6) and the like. The human p53 gene is not limited to the example and may be a homolog (an ortholog or a paralog) thereof or a mutant thereof. The sequence information of p53 genes that other mammals have can also be obtained from a known database such as GenBank.

The suppression of the activity of p53 can be achieved, for example, by the presence of an inhibitor of the p53 activity or by inhibition of the expression of p53 gene. For example, when the intracellular amount of an inhibitor of the p53 activity is higher than that in the second/third-trimester trophoblast cells, it can be determined that the activity of p53 is suppressed in the cells compared to that in the second/third-trimester trophoblast cells. Moreover, for example, when the intracellular mRNA amount or protein amount of p53 is lower than that in the second/third-trimester trophoblast cells, it can be determined that the activity of p53 is suppressed in the cells compared to that in the second/third-trimester trophoblast cells.

The inhibitor of the p53 activity is not particularly limited, but an example thereof is dominant negative of p53. The dominant negative of p53 is not particularly limited as long as the dominant negative is a polypeptide which functions dominantly over p53 and which can inhibit the function of p53. An example of the p53 dominant negative is a truncated protein in which a part of p53 is deleted. As long as the P53 dominant negative binds to the ligand of p53 and inhibits the function of p53, the deleted amino acid sequence is not particularly limited. For example, in the P53 dominant negative, the N-terminal of p53 may be deleted, or the C-terminal may be deleted. A specific example of human p53 dominant negative is, for example, a polypeptide containing the amino acid sequence from the 300th to the 393rd positions of the amino acid sequence of SEQ ID NO: 6 (SEQ ID NO: 8).

The p53 dominant negative may be introduced into the cells after linking a cell-penetrating peptide or the like. In this case, the p53 dominant negative linked to the cell-penetrating peptide may be in the cells. Alternatively, an exogenous p53 dominant negative gene functionally linked to a second exogenous inducible promoter may be introduced into the cells to induce the expression of the exogenous p53 dominant negative gene in the cells. In this case, the TS-like cells of the embodiment contains an exogenous p53 dominant negative gene functionally linked to a second exogenous inducible promoter. The p53 dominant negative gene may be in the genome (for example, the nuclear genome) or may be outside the genome as a plasmid or the like.

When the TS-like cells of the embodiment contains an exogenous p53 dominant negative gene functionally linked to a second exogenous inducible promoter, the second exogenous inducible promoter is not particularly limited. The second exogenous inducible promoter may be a promotor similar to those listed as the first exogenous inducible promoter. The second exogenous inducible promoter may be the same as or different from the first exogenous inducible promoter. The second exogenous inducible promoter is preferably the same as the first exogenous inducible promoter because the transcriptional activity can be induced with the same inducing factor as that for the first exogenous inducible promoter.

Examples of the exogenous p53 dominant negative gene functionally linked to the second exogenous inducible promoter include (A) to (G) below.

(A) A polynucleotide which is a truncated gene of a wild-type p53 gene (for example, SEQ ID NO: 5) and which encodes a protein that functions as p53 dominant negative (for example, SEQ ID NO: 7).

(B) A polynucleotide having a nucleotide sequence encoding a protein which is a truncated protein (for example, SEQ ID NO: 8) of a wild-type p53 protein (for example, SEQ ID NO: 6) and which functions as p53 dominant negative.

(C) A polynucleotide encoding a protein which has an amino acid sequence of a truncated protein (for example, SEQ ID NO: 8) of a wild-type p53 protein (for example, SEQ ID NO: 6) having mutation of one amino acid or a plurality of amino acids and which functions as p53 dominant negative.

(D) A polynucleotide encoding a protein which has an amino acid sequence having a sequence identity of 70% or more with the amino acid sequence of a truncated protein (for example, SEQ ID NO: 8) of a wild-type p53 protein (for example, SEQ ID NO: 6) and which functions as p53 dominant negative.

(E) A polynucleotide which has a nucleotide sequence of a truncated gene (for example, SEQ ID NO: 7) of a wild-type p53 gene (for example, SEQ ID NO: 5) having mutation of one nucleotide or a plurality of nucleotides and which encodes a protein that functions as p53 dominant negative.

(F) A polynucleotide which has a nucleotide sequence having a sequence identity of 70% or more with a truncated gene (for example, SEQ ID NO: 7) of a wild-type p53 gene (for example, SEQ ID NO: 5) and which encodes a protein that functions as p53 dominant negative.

(G) A polynucleotide which hybridizes to a truncated gene (for example, SEQ ID NO: 7) of a wild-type p53 gene (for example, SEQ ID NO: 5) under stringent conditions and which encodes a protein that functions as p53 dominant negative.

In (C) and (E) above, the "mutation" may be any of deletion, substitution, addition and insertion or a combination thereof.

In (C) above, the "plurality" is not particularly limited but is, for example, 2 to 10 residues, 2 to 90 residues, 2 to 8 residues, 2 to 7 residues, 2 to 6 residues, 2 to 5 residues, 2 to 4 residues, 2 or 3 residues or 2 residues.

In (E) above, the "plurality" is not particularly limited but is, for example, 2 to 30 residues, 2 to 20 residues, 2 to 15 residues, 2 to 10 residues, 2 to 9 residues, 2 to 8 residues, 2 to 7 residues, 2 to 6 residues, 2 to 5 residues, 2 to 4 residues, 2 or 3 residues or 2 residues.

In (D) and (F) above, the sequence identity is not particularly limited as long as the sequence identity is 70% or more, but the sequence identity is 80% or more, 85% or more, 90% or more, 95% or more, 96% or more, 97% or more, 98% or more or 99% or more.

The "stringent conditions" in (G) above are similar to those described above.

In (B) to (E) above, as the degenerate codons, codons with high usage in the cells of the placental mammal used are preferably used. For example, when used for a human second/third-trimester trophoblast cells, codons with high usage in human cells are preferably used. That is, the codons are preferably optimized for human codons.

When the TS-like cell of the embodiment contains the exogenous p53 dominant negative gene functionally linked to the second exogenous inducible promoter, the expression state of the exogenous p53 dominant negative is not particularly limited. The TS-like cell of the embodiment may be a cell in which the exogenous p53 dominant negative gene is not expressed and in which the exogenous p53 dominant negative is not detected. In this case, the TS-like cell of the embodiment may be a cell in which the activity of p53 is not suppressed compared to that in the second/third-trimester trophoblast cells.

The inhibition of the transcription of the p53 gene can be achieved, for example, with a small interfering nucleic acid such as siRNA. The "small interfering nucleic acid" refers to a small nucleic acid of around 18 to 25 base pairs which suppresses the expression of the target gene by RNA interference. In the small interfering nucleic acid, one nucleic acid molecule may form the base pairs by a hairpin structure, or two nucleic acid molecules may form the base pairs. The small interfering nucleic acid may consist of RNA or may contain RNA and DNA. The small interfering nucleic acid which suppresses the expression of the p53 gene can be designed based on a known technique.

The small interfering nucleic acid for the p53 gene may be introduced into the cells using a transfection reagent or the like. In this case, the small interfering nucleic acid for the p53 gene may be in the cells. Alternatively, a gene encoding the small interfering nucleic acid for the p53 gene functionally linked to a second exogenous inducible promoter may be introduced into the cells to induce the expression of the small interfering nucleic acid in the cells. In this case, the TS-like cell of the embodiment contains a gene encoding the small interfering nucleic acid for the p53 gene functionally linked to a second exogenous inducible promoter. As the second exogenous inducible promoter, a promoter similar to that for the p53 dominant negative gene can be used. When the TS-like cell of the embodiment contains a gene encoding the small interfering nucleic acid for the p53 gene functionally linked to a second exogenous inducible promoter, the expression state of the gene is not particularly limited. The TS-like cell of the embodiment may be a cell in which the gene is not expressed and in which the small interfering nucleic acid for the p53 gene is not detected. In this case, the TS-like cell of the embodiment may be a cell in which the activity of p53 is not suppressed compared to that in the second/third-trimester trophoblast cells.

The inhibition of the transcription of the p53 gene may be achieved, for example, with an antisense nucleic acid such as antisense RNA. The "antisense nucleic acid" refers to a nucleic acid which hybridizes to the mRNA of the target gene and which suppresses the expression of the target gene. The antisense nucleic acid may consist of RNA or may contain RNA and DNA. The antisense nucleic acid which suppresses the expression of the p53 gene can be designed based on a known technique.

The antisense nucleic acid for the p53 gene may be introduced into the cells using a transfection reagent or the like. In this case, the antisense nucleic acid for the p53 gene may be in the cells. Alternatively, a gene encoding the antisense nucleic acid for the p53 gene functionally linked to a second exogenous inducible promoter may be introduced into the cells to induce the expression of the antisense nucleic acid in the cells. In this case, the TS-like cell of the embodiment contains a gene encoding the antisense nucleic acid for the p53 gene functionally linked to a second exogenous inducible promoter. As the second exogenous inducible promoter, a promoter similar to that for the p53 dominant negative gene can be used. When the TS-like cell of the embodiment contains a gene encoding the antisense nucleic acid for the p53 gene functionally linked to a second exogenous inducible promoter, the expression state of the gene is not particularly limited. The TS-like cell of the embodiment may be a cell in which the gene is not expressed and in which the antisense nucleic acid for the p53 gene is not detected. In this case, the TS-like cell of the embodiment may be a cell in which the activity of p53 is not suppressed compared to that in the second/third-trimester trophoblast cells.

(Feature (b): Promotion of MYC Gene Expression)

In the TS-like cell of the embodiment, the expression of a MYC gene may be promoted compared to that in the second/third-trimester trophoblast cells. Here, that "the expression is promoted compared to that in the second/third-trimester trophoblast cells" includes that the expression is detected in the cells when the expression is not detected in the second/third-trimester trophoblast cells and that an exogenous gene which the second/third-trimester trophoblast cells does not contain is introduced and expressed.

MYC is a nuclear phosphoprotein encoded by a protooncogene and involves in progress of cell cycle, apoptosis and cell transformation. The sequence of a MYC gene is known and can be obtained from a known database such as GenBank. Examples of human MYC (NCBI Gene ID:4609) include those registered with NM_001354870.1 (SEQ ID NOs: 9 and 10) or NM_002467.6 (SEQ ID NOs: 11 and 12) and the like. The human MYC gene is not limited to the examples and may be a homolog (an ortholog or a paralog) thereof or a mutant thereof. The sequence information of MYC genes that other mammals have can be obtained from a known database such as GenBank.

The promotion of the expression of a MYC gene can be achieved, for example, by introducing a MYC gene functionally linked to a second exogenous inducible promoter and inducing the expression of the MYC gene. In this case, the cell contains an exogenous MYC gene functionally linked to a second exogenous inducible promoter.

When the TS-like cell of the embodiment contains an exogenous MYC gene functionally linked to a second exogenous inducible promoter, the second exogenous inducible promoter is not particularly limited. As the second exogenous inducible promoter, a promoter similar to that for the p53 dominant negative gene can be used. When the TS-like cell of the embodiment contains an exogenous MYC gene functionally linked to a second exogenous inducible promoter, the exogenous MYC gene is preferably in the expressed state.

The TS-like cell of the embodiment preferably contains an exogenous SALL4 gene functionally linked to a first exogenous inducible promoter and an exogenous p53 dominant negative gene functionally linked to a second exogenous inducible promoter. The first exogenous inducible promoter and the second exogenous inducible promoter are preferably same inducible promoters because the transcriptional activity can be induced with a same inducing factor. As the first exogenous inducible promoter and the second exogenous inducible promoter, for example, a tetracycline-inducible promoter can be used, but the promoters are not limited thereto.

The TS-like cell of the embodiment can be produced by the method for producing a TS-like cell described below. The TS-like cell of the embodiment has potential to differentiate into placenta constituting cells and thus can be differentiated into an EVT, an ST or the like. Accordingly, the TS-like cells can be used as a research material for basic research on early development of placental mammals, analysis of placental functions and the like. Moreover, the TS-like cell of the embodiment is induced from a trophoblast cell derived from a placenta of or after the second trimester of pregnancy, in which a pregnancy-associated disease becomes evident. TS-like cells induced from a second/third-trimester trophoblast cells derived from an individual which has developed a disease can be used for elucidating the pathological conditions of the pregnancy-associated disease and developing a therapeutic method. Moreover, application for reproductive technique and regenerative medicine is also expected.

[Production Method of Trophoblast Stem Cell-Like Cells (TS-Like Cells)]

In an embodiment, the invention provides a method for producing a trophoblast stem cell-like cell (TS-like cell) having potential to differentiate into a placenta-constituting cell. The method for producing a TS-like cell of the embodiment includes the steps of (i) to (iii) below.

(i) A step of preparing a trophoblast cell derived from a placenta of or after the second trimester of pregnancy.
(ii) A step of inducing the expression of a SALL4 gene in the trophoblast cell.
(iii) A step of conducting at least one selected from the group consisting of (A) and (B) below:

(A) suppressing the activity of p53 of the trophoblast cell; and
(B) inducing the expression of a MYC gene in the trophoblast cell.

<Step (i)>

The step (i) is a step of preparing trophoblast cells derived from a placenta of or after the second trimester of pregnancy.

The trophoblast cells derived from a placenta of or after the second trimester of pregnancy (second/third-trimester trophoblast cells) is similar to that explained in the section "<Trophoblast Cells>" in "[Trophoblast Stem Cell-Like Cells (TS-Like Cells)]" above. The second/third-trimester trophoblast cells can be isolated from a placenta of or after the second trimester of pregnancy by a known method. The method for isolating the second/third-trimester trophoblast cells may be the method explained in the section "<Trophoblast Cells>" above.

<Step (ii)>

The step (ii) is a step of inducing the expression of a SALL4 gene in the second/third-trimester trophoblast cells.

The method for inducing the expression of a SALL4 gene in the second/third-trimester trophoblast cells is not particularly limited, and a known method can be used.

An example of the method for inducing the expression of a SALL4 gene is a method including (ii-1) and (ii-2) below.

(ii-1) Introducing a polynucleotide containing an exogenous SALL4 gene functionally linked to a first exogenous inducible promoter into the second/third-trimester trophoblast cells or
introducing a polynucleotide containing the first exogenous inducible promoter into the second/third-trimester trophoblast cells at the upstream of an endogenous SALL4 gene in such a manner that the endogenous SALL4 gene is functionally linked to the first exogenous inducible promoter.
(ii-2) Inducing the expression of the exogenous SALL4 gene or the endogenous SALL4 gene with a first inducing factor which induces the transcriptional activity of the first exogenous inducible promoter.

(ii-1)

(ii-1) may be an operation of introducing a polynucleotide containing an exogenous SALL4 gene functionally linked to a first exogenous inducible promoter into the second/third-trimester trophoblast cells. The SALL4 gene functionally linked to the first exogenous inducible promoter is similar to that explained in the section "[Trophoblast Stem Cell-Like Cells (TS-Like Cells)]" above.

The method for introducing a polynucleotide containing the exogenous SALL4 gene functionally linked to the first exogenous inducible promoter into the second/third-trimester trophoblast cells is not particularly limited, and a known method can be used. For example, the exogenous SALL4 gene functionally linked to the first exogenous inducible promoter may be cloned in an appropriate expression vector that can be expressed in the second/third-trimester trophoblast cells as the host cells, and the expression vector may be introduced into the second/third-trimester trophoblast cells.

The expression vector may contain, in addition to the polynucleotide containing the exogenous SALL4 gene functionally linked to the first exogenous inducible promoter, an enhancer, a poly(A) addition signal, a marker gene, an origin of replication, a gene encoding a protein which binds to the origin of replication and regulates replication and the like according to the need. The "marker gene" refers to a gene which enables screening and selection of cells when the marker gene is introduced into the cells. Examples of the marker gene include a drug-resistant gene, a fluorescent protein gene, a luminescent enzyme gene, a chromogenic enzyme gene and the like, but the marker gene is not limited thereto. A kind of marker gene may be used alone, or two or more kinds may be used in combination. Examples of the drug-resistant gene include a neomycin-resistant gene, a tetracycline-resistant gene, a kanamycin-resistant gene, a zeocin-resistant gene, a hygromycin-resistant gene, a puromycin-resistant gene and the like, but the drug-resistant gene is not limited thereto. Examples of the fluorescent protein gene include a green fluorescent protein (GFP) gene, a yellow fluorescent protein (YFP) gene, a red fluorescent protein (RFP) gene and the like, but the fluorescent protein gene is not limited thereto. Examples of the luminescent enzyme gene include a luciferase gene and the like, but the luminescent enzyme gene is not limited thereto. Examples of the chromogenic enzyme gene include β-galactosidase gene, β-glucuronidase gene, alkaline phosphatase gene and the like, but the chromogenic enzyme gene is not limited thereto.

The kind of the expression vector is not particularly limited, and a known expression vector can be used. Examples of the expression vector include an episomal vector, an artificial chromosome vector, a plasmid vector, a viral vector and the like.

An example of the episomal vector is a vector containing sequences necessary for autonomous replication derived from EBV, SV40 or the like as vector elements. The vector elements necessary for autonomous replication are specifically an origin of replication and a gene encoding a protein which binds to the origin of replication and regulates replication, and examples thereof are an origin of replication, oriP, and EBNA-1 gene for EBV and an origin of replication, ori, and SV40LT gene for SV40.

The artificial chromosome vector may be YAC (Yeast artificial chromosome) vector, BAC (Bacterial artificial chromosome) vector, PAC (P1-derived artificial chromosome) vector or the like.

The plasmid vector is not particularly limited as long as the plasmid vector can be expressed in pluripotent stem cells as the target of introduction. Examples of a plasmid vector for expression in animal cells include pA1-11, pXT1, pRc/CMV, pRc/RSV, pcDNAI/Neo and the like.

The viral vector may be a retroviral (including lentiviral) vector, an adenoviral vector, an adeno-associated viral vector, a Sendai viral vector, a herpesviral vector, a vaccinia viral vector, a poxviral vector, a polioviral vector, a shirubisu viral vector, a rhabdoviral vector, a paramyxoviral vector, an orthomyxoviral vector or the like.

The method for introducing the expression vector into the second/third-trimester trophoblast cells is not particularly limited and can be appropriately selected depending on the kind of the expression vector. Examples of the method for introducing the expression vector into the second/third-trimester trophoblast cells include lipofection, microinjection, DEAE-dextran method, gene gun method, electroporation, calcium phosphate method and the like. When the expression vector is a viral vector, an example of the method for infecting the second/third-trimester trophoblast cells with the viral vector is polybrene method.

When the expression vector contains an antibiotic-resistant gene as a selection marker, cells into which the expression vector has been introduced can be selected efficiently by culturing the cells in a medium containing the antibiotic corresponding to the antibiotic-resistant gene after introducing the expression vector.

(ii-1) may be an operation of introducing a polynucleotide containing a first exogenous inducible promoter upstream of an endogenous SALL4 gene in such a manner that the endogenous SALL4 gene is functionally linked to the first exogenous inducible promoter. The first exogenous inducible promoter is similar to that described above.

The method for introducing a polynucleotide containing a first exogenous inducible promoter upstream of an endogenous SALL4 gene is not particularly limited, and a known site-specific introduction method can be used. An example of the method for site-specific introduction of a polynucleotide is a genome editing technique using a site-specific nuclease. The genome editing technique may be CRISPR/Cas system, a zinc finger nuclease (ZFN), TALEN or the like. A method for site-specific insertion of a desired polynucleotide using such a genome editing technique is known. For example, when CRISPR/Cas system is used, the protospacer adjacent motif (PAM) sequence (NGG for CRISPR/Cas9 system of *S. pyogenes*) at the upstream of the endogenous SALL4 gene is searched, and gRNA can be designed using around upstream 20 bases adjacent to the PAM sequence as the target sequence. The gRNA may be designed using known gRNA design software or the like.

(ii-2)

(ii-2) is an operation of inducing the expression of the exogenous SALL4 gene or the endogenous SALL4 gene with a first inducing factor which induces the transcriptional activity of the first exogenous inducible promoter.

The first inducing factor is a factor which induces the transcriptional activity of the first exogenous inducible promoter. The first inducing factor can be appropriately selected depending on the kind of the first exogenous inducible promoter. For example, when the first exogenous inducible promoter is a tetracycline-inducible promoter, a tetracycline or an analog thereof can be used as the first inducing factor. By culturing the cells after (ii-1) in a medium containing the first inducing factor, the transcriptional activity of the first exogenous inducible promoter is induced. As a result, the expression of the exogenous SALL4 gene or the endogenous SALL4 gene functionally linked to the first exogenous inducible promoter can be induced.

The period for inducing the expression of the SALL4 gene in the step (ii) is not particularly limited. The period for inducing the expression of the SALL4 gene can be, for example, five days or longer, eight days or longer, 10 days or longer, 12 days or longer, 15 days or longer, 18 days or longer or 20 days or longer. By inducing the expression of the SALL4 gene for the period of the lower limit or longer, homogeneous TS-like cells having potential to differentiate into placenta constituting cells can be obtained. The upper limit of the period for inducing the expression of the SALL4 gene is not particularly limited. For example, the cells may be cultured in a medium containing the first inducing factor until just before inducing into placenta constituting cells (an EVT, an ST or the like), and the expression of the SALL4 gene may be induced. Alternatively, the induced expression of the SALL4 gene may be stopped after TS-like cells is induced. Once a TS-like cell is induced, the TS-like cells can be maintained while keeping the potential to differentiate into placenta constituting cells even without inducing the expression of the SALL4 gene. Thus, the period for inducing the expression of the SALL4 gene may be, for example, 50 days or shorter, 40 days or shorter, 30 days or shorter or 25 days or shorter.

The period for inducing the expression of the SALL4 gene can be regulated with the presence of the first inducing factor in the medium for culturing the cells.

<Step (iii)>

The step (iii) is a step of conducting at least one selected from the group consisting of (A) and (B) below.

    (A) Suppressing the activity of p53 of the second/third-trimester trophoblast cells.
    (B) Inducing the expression of a MYC gene in the second/third-trimester trophoblast cells.

    (A)

    (A) is a step of suppressing the activity of p53 of the second/third-trimester trophoblast cells. The method for suppressing the activity of p53 of the second/third-trimester trophoblast cells is not particularly limited, and a known method can be used. Examples of the method for suppressing the activity of p53 include a method for causing an inhibitor of the p53 activity to exist in the second/third-trimester trophoblast cells, a method for suppressing the expression of the p53 gene of the second/third-trimester trophoblast cells and the like.

An example of the inhibitor of the p53 activity is dominant negative of p53. The p53 dominant negative is similar to that explained in the section "Trophoblast Stem Cell-Like Cells (TS-Like Cells)]" above. The p53 dominant negative may be introduced into the cells after linking a cell-penetrating peptide or the like. Alternatively, an exogenous p53 dominant negative gene functionally linked to a second exogenous inducible promoter may be introduced into the cells. The exogenous p53 dominant negative gene functionally linked to the second exogenous inducible promoter is similar to that explained in the section "Trophoblast Stem Cell-Like Cells (TS-Like Cells)]" above.

When an exogenous p53 dominant negative gene functionally linked to a second exogenous inducible promoter is used, (A) may include (A-1) and (A-2) below.

(A-1) Introducing a polynucleotide containing an exogenous p53 dominant negative gene functionally linked to a second exogenous inducible promoter into the second/third-trimester trophoblast cells.
(A-2) Inducing the expression of the exogenous p53 dominant negative gene with a second inducing factor which induces the transcriptional activity of the second exogenous inducible promoter.

(A-1) is an operation of introducing a polynucleotide containing an exogenous p53 dominant negative gene functionally linked to a second exogenous inducible promoter into the second/third-trimester trophoblast cells. As the method for introducing the polynucleotide into the second/third-trimester trophoblast cells, a method similar to the method for introducing the polynucleotide containing the exogenous SALL4 gene functionally linked to the first exogenous inducible promoter can be used. The second exogenous inducible promoter may be the same as or different from the first exogenous inducible promoter. The second exogenous inducible promoter is preferably the same as the first exogenous inducible promoter because the transcriptional activity can be induced with the same inducing factor as that for the first exogenous inducible promoter.

(A-2) is an operation of inducing the expression of the exogenous p53 dominant negative gene with a second inducing factor which induces the transcriptional activity of the second exogenous inducible promoter.

The second inducing factor is a factor which induces the transcriptional activity of the second exogenous inducible promoter. The second inducing factor can be appropriately selected depending on the kind of the second exogenous inducible promoter. For example, when the second exogenous inducible promoter is a tetracycline-inducible promoter, a tetracycline or an analog thereof can be used as the second inducing factor. By culturing the cells after (A-1) in a medium containing the second inducing factor, the transcriptional activity of the second exogenous inducible promoter is induced. As a result, the expression of the exogenous p53 dominant negative gene functionally linked to the second exogenous inducible promoter can be induced. When the second exogenous inducible promoter is the same as the first exogenous inducible promoter, the same inducing factor as the first inducing factor can be used as the second inducing factor.

(ii-1) and (A-1) may be conducted simultaneously or separately. Because the introduction operation can be conducted at a time, (ii-1) and (A-1) are preferably conducted simultaneously. In this case, the expression vector used in (ii-1) and the expression vector used in (A-1) can be mixed and used for the introduction operation into the second/third-trimester trophoblast cells.

(ii-2) and (A-2) are preferably conducted simultaneously. By conducting (ii-2) and (A-2) simultaneously, the expression of the SALL4 gene and the p53 dominant negative gene can be induced simultaneously. By causing SALL4 and p53 dominant negative to act simultaneously, the second/third-trimester trophoblast cells can be induced into TS-like cells efficiently.

The method for suppressing the activity of p53 of the second/third-trimester trophoblast cells may be a method for suppressing the expression of the p53 gene of the second/third-trimester trophoblast cells. In this case, a small interfering nucleic acid or an antisense nucleic acid for the p53 gene may be used. The small interfering nucleic acid and the antisense nucleic acid for the p53 gene are similar to those explained in the section "Trophoblast Stem Cell-Like Cells (TS-Like Cells)]" above. The small interfering nucleic acid and the antisense nucleic acid for the p53 gene may be introduced into the second/third-trimester trophoblast cells using a transfection reagent or the like.

Alternatively, a polynucleotide containing a gene encoding the small interfering or antisense nucleic acid for the p53 gene functionally linked to a second exogenous inducible promoter may be introduced into the second/third-trimester trophoblast cells. As the method for introducing the polynucleotide into the second/third-trimester trophoblast cells, a method similar to the method for introducing the polynucleotide containing the exogenous SALL4 gene functionally linked to the first exogenous inducible promoter can be used.

By culturing the cells after the introduction of the polynucleotide in a medium containing the second inducing factor, the expression of the small interfering nucleic acid or the antisense nucleic acid for the p53 gene can be induced.

The period for suppressing the activity of p53 in (A) is not particularly limited. The period for suppressing the activity of p53 can be, for example, five days or longer, eight days or longer, 10 days or longer, 12 days or longer, 15 days or longer, 18 days or longer or 20 days or longer. By suppressing the activity of p53 for the period of the lower limit or longer, homogeneous TS-like cells having potential to differentiate into placenta constituting cells can be obtained. The upper limit of the period for suppressing the activity of p52 is not particularly limited. For example, the cells may be cultured in a medium containing the second inducing factor until just before inducing into placenta constituting cells (an EVT, an ST or the like), and the expression of the p53 dominant negative gene, the small interfering nucleic acid gene or the antisense nucleic acid gene may be induced. Alternatively, the suppression of the activity of p53 may be stopped, after TS-like cells are induced. Once a TS-like cell is induced, the TS-like cells can be maintained while keeping the potential to differentiate into placenta constituting cells even without the suppression of the activity of p53. Thus, the period for suppressing the activity of p53 may be, for example, 50 days or shorter, 40 days or shorter, 30 days or shorter or 25 days or shorter.

The period for suppressing the activity of p53 can be regulated, for example, with the presence of the second inducing factor in the medium for culturing the cells. The period for suppressing the activity of p53 may be the same as or different from the period for inducing the expression of the SALL4 gene but is preferably the same.

(B)

(B) is a step of inducing the expression of a MYC gene in the second/third-trimester trophoblast cells. The method for inducing the expression of a MYC gene in the second/third-trimester trophoblast cells is not particularly limited, and a known method can be used. An example of the method for inducing the expression of a MYC gene is a method including (B-1) and (B-2) below.

(B-1) Introducing an exogenous MYC gene functionally linked to a second exogenous inducible promoter.

(B-2) Inducing the expression of the exogenous MYC gene with a second inducing factor which induces the transcriptional activity of the second exogenous inducible promoter.

(B-1) can be conducted in a similar manner to that in (A-1) above except that the gene functionally linked to the second exogenous inducible promoter is an exogenous MYC gene. The exogenous MYC gene is similar to that explained in the section "Trophoblast Stem Cell-Like Cells (TS-Like Cells)" above.

(B-2) can be conducted in a similar manner to that in (A-2) above except that the gene functionally linked to the second exogenous inducible promoter is an exogenous MYC gene. In this regard, however, the induced expression of the exogenous MYC gene is preferably continuously maintained after starting inducing the expression. By continuously maintaining the induced expression of the exogenous MYC gene, the TS-like cells can be maintained while keeping the potential to differentiate into placenta constituting cells.

<Medium>

An example of the medium for culturing the cells in the step (ii) and the step (iii) is a medium containing a growth factor and a ROCK inhibitor. The medium can be prepared, for example, by adding a growth factor and a ROCK inhibitor to a medium generally used for culturing animal cells as the basal medium. Examples of the basal medium include Doulbecco's modified Eagle's Medium (DMEM), DMEM/F12 medium, IMDM medium, Medium199 medium, Eagle's Minimum Essential Medium (EMEM), αMEM medium, Ham's F12 medium, RPMI1640 medium, Fischer's medium, mixture media thereof and the like. A preferable basal medium is, for example, DMEM/F12.

The growth factor is not particularly limited, but examples thereof include epidermal growth factor (EGF), insulin, transforming growth factor (TGF) and the like. As the growth factor, commercial products can be used. In particular, the growth factor is preferably EGF.

The concentration of the growth factor in the medium is not particularly limited but can be, for example, 10 to 100 ng/mL and is preferably 20 to 80 ng/mL, more preferably 30 to 70 ng/mL, further preferably 40 to 60 ng/mL.

The ROCK (Rho associated coiled-coil containing protein kinase: Rho-binding kinase) inhibitor is not particularly limited as long as the function of Rho-binding kinase can be suppressed. Examples of the ROCK inhibitor include trans-N-(4-pyridyl)-4-(1-aminoethyl)-cyclohexanecarboxamide, 1-(5-isoquinolinylsulfonyl)homopiperazine, salts thereof and the like. Moreover, examples thereof include small molecule inhibitors such as Fasudil/HA1077, H-1152 and Wf-536, derivatives thereof and the like. Examples thereof also include an antisense nucleic acid, siRNA and a dominant negative mutant of ROCK, expression vectors thereof and the like.

Commercial products of trans-N-(4-pyridyl)-4-(1-aminoethyl)-cyclohexanecarboxamide or a salt thereof are Y27632 ((R)-(+)-trans-N-(4-pyridyl)-4-(1-aminoethyl)-cyclohexanecarboxamide·2HCl·H$_2$O) and the like. A kind of ROCK inhibitor may be used alone, or two or more kinds may be used in combination.

As the ROCK inhibitor, Y27632 is preferably used.

The concentration of the ROCK inhibitor in the medium is not particularly limited but can be, for example, 0.1 to 50 μM and is preferably 1 to 20 μM, more preferably 1 to 10 μM, further preferably 3 to 8 μM.

The medium preferably contains at least one selected from the group consisting of an ALK5 inhibitor and a GSK3β inhibitor in addition to the growth factor and the ROCK inhibitor.

The ALK5 inhibitor is not particularly limited as long as the function of ALK5 can be suppressed. Examples of the ALK5 inhibitor include 4-[4-(1,3-benzodioxol-5-yl)-5-(2-pyridyl)-1H-imidazol-2-yl]benzamide, a salt thereof and the like. Examples thereof also include small molecule inhibitors such as A83-01 (3-(6-methylpyridin-2-yl)-N-phenyl-4-quinolin-4-ylpyrazole-1-carbothioamide), 2-(3-(6-methylpyridin-2-yl)-1H-pyrazol-4-yl)-1,5-naphthyridine, Wnt3a/BIO, GW788388, SM16, IN-1130, GW6604, SB-505124 and a pyrimidine derivative. Examples thereof also include an antisense nucleic acid, siRNA and a dominant negative mutant of ALK5, expression vectors thereof and the like. Commercial products of 4-[4-(1,3-benzodioxol-5-yl)-5-(2-pyridyl)-1H-imidazol-2-yl]benzamide or a salt thereof are SB431542 and the like. A kind of ALK5 inhibitor may be used alone, or two or more kinds may be used in combination.

As the ALK5 inhibitor, SB431542 or A83-01 is preferably used, and both SB431542 and A83-01 are preferably used.

The concentration of the ALK5 inhibitor in the medium is not particularly limited but can be, for example, 0.1 to 20 μM and is preferably 0.2 to 10 μM, more preferably 0.5 to 5 μM, further preferably 0.5 to 3 μM. When SB431542 is used as the ALK5 inhibitor, the concentration of SB431542 in the medium can be, for example, 0.1 to 10 μM and is preferably 0.2 to 5 μM, more preferably 0.5 to 3 μM, further preferably 0.7 to 2 μM. When A83-01 is used as the ALK5 inhibitor, the concentration of A83-01 in the medium can be, for example, 0.1 to 5 μM and is preferably 0.2 to 3 μM, more preferably 0.3 to 2 μM, further preferably 0.3 to 1 μM.

The GSK3β inhibitor is not particularly limited as long as the function of GSK3β can be suppressed. Examples of the GSK3β inhibitor include 6-[[2-[[4-(2,4-dichlorophenyl)-5-(4-methyl-1H-imidazol-2-yl)-2-pyrimidinyl]amino]ethyl]amino]nicotinonitrile and the like. Examples thereof also include small molecule inhibitors such as Kenpaullone, 1-Azakenpaullone, CHIR98014, AR-A014418, CT99021, CT20026, SB216763, AR-A014418, lithium, SB415286, TDZD-8, BIO, BIO-acetoxime, (5-methyl-1H-pyrazol-3-yl)-(2-phenylquinazolin-4-yl)amine, pyridocarbazole-cyclopentadienylruthenium (cyclopenadienylruthenium) complex, TDZD-8 4-benzyl-2-methyl-1,2,4-thiadiazolidine-3,5-dione, 2-thio(3-iodobenzyl)-5-(1-pyridyl)-[1,3,4]-oxadiazole, OTDZT, alpha-4-dibromoacetophenone, AR-AO 144-18, 3-(1-(3-hydroxypropyl)-1H-pyrrolo[2,3-b]pyridin-3-yl]-4-pyrazin-2-yl-pyrrole-2,5-dione; TWSI 19 pyrrolopyrimidine compound, L803 H-KEAPPAPPQSpP-NH2 or a myristoylated form thereof: 2-chloro-1-(4,5-dibromo-thiophen-2-yl)-ethanone, SB216763 and SB415286. Examples thereof also include an antisense nucleic acid, siRNA and a dominant negative mutant of GSK3β, expression vectors thereof and the like. Commercial products of 6-[[2-[[4-(2,4-dichlorophenyl)-5-(4-methyl-1H-imidazol-2-yl)-2-pyrimidinyl]amino]ethyl]amino]nicotinonitrile are CHIR99021 and the like. A kind of GSK3β inhibitor may be used alone, or two or more kinds may be used in combination.

As the GSK3β inhibitor, CHIR99021 is preferably used.

The concentration of the GSK3β inhibitor in the medium is not particularly limited but can be, for example, 0.1 to 20 μM and is preferably 0.2 to 10 μM, more preferably 0.5 to 5 μM, further preferably 0.5 to 3 μM.

The medium may further contain a histone deacetylase (HDAC) inhibitor in addition to the above components.

The HDAC inhibitor is not particularly limited as long as the function of HDAC can be suppressed. Examples of the HDAC inhibitor include small molecule inhibitors such as valproic acid (VPA) trichostatin A, sodium butyrate, MC1293 and M344. Examples thereof also include an antisense nucleic acid, siRNA and a dominant negative mutant of HDAC, expression vectors thereof and the like. A kind of HDAC inhibitor may be used alone, or two or more kinds may be used in combination.

As the HDAC inhibitor, VPA is preferably used.

The concentration of the HDAC inhibitor in the medium is not particularly limited but can be, for example, 0.01 to 10 mM and is preferably 0.1 to 5 mM, more preferably 0.5 to 2 mM, further preferably 0.5 to 1 mM.

Another component may be added to the basal medium according to the need in addition to the above components. Examples of the other component include serum (fetal bovine serum (FBS) or the like) or one or more serum replacements such as albumin, transferrin, sodium selenite, ITS-X (Invitrogen), knockout serum replacements ((KSR); a serum replacement of FBS for culturing ES cells), N2 supplement (Invitrogen), B27 supplement (Invitrogen), fatty acids, insulin, collagen precursors, trace elements, 2-mercaptoethanol and 3'-thiolglycerol. Examples thereof also include one or more substances selected from lipid, amino acids, L-glutamine, Glutamax, nonessential amino acids, vitamins, growth factors, antibiotics, antioxidants, pyruvic acid, buffers and inorganic salts. Preferable other components are FBS, bovine serum albumin (BSA), ITS-X, L-ascorbic acid, 2-mercaptomethanol and antibiotics (penicillin, streptomycin and the like).

Specific examples of the preferable medium include the Term-1 medium and the Term-2 medium described in the Examples. When the transcriptional activity of the first exogenous inducible promoter or the second exogenous inducible promoter is induced, the medium to which the first inducing factor or the second inducing factor has been added may be used.

The production method of the embodiment can be used for producing the TS-like cells of the embodiment.

Examples

Although the invention is explained with the Examples below, the invention is not limited to the Examples below.

1. Isolation of CT Cells from Placenta of or after Second Trimester of Pregnancy

1-1. Reagents and Instruments

The reagents and the instruments used for the test are shown in Table 1.

[Table 1]

| Reagent/Instrument | Supplier | Cat# |
|---|---|---|
| Accumax | Innovative Cell Technologies | #AM105 |
| TrypLE | Thermo Fisher Scientific | #12605028 |
| FBS | GIBCO | #16141-079 |
| PBS | Wako | #166-23555 |
| DMEM | GIBCO | #11995-065 |
| 10× HBSS | GIBCO | #14185 |
| TrypLE | GIBCO | #12604-021 |
| Sodium Chloride | Wako | #191-01665 |
| Percoll | GE Healthcare | #17-5445-02 |
| 500 mM EDTA | GIBCO | #15575 |
| anti-CD49f antibody | PE conjugated, Miltenyi Biotec | #130-097-246 |
| PE selection kit | STEMCELL | #18551 |
| Magnet | STEMCELL | #18001 |
| 14 ml tube | BD Falcon | #352057 |
| 70 mm Cell Strainer | BD Falcon | #352350 |

1-2. Preparation of Medium

Reagents and a medium were prepared as follows.

By dissolving 9 g of sodium chloride in 1000 mL of water, physiological saline (0.9% sodium chloride) was prepared. The physiological saline was autoclaved and stored at room temperature until use.

By mixing 196 mL of PBS, 400 μL of 500 mM EDTA and 4 mL of FBS, PBS-EDTA was prepared. The PBS-EDTA was stored at 4°C until use.

By mixing 160 mL of PBS, 36 mL of DMEM and 4 mL of FBS, PBS-DMEM was prepared. The PBS-DMEM was stored at 4°C until use.

By mixing 35 mL of Percoll, 5 mL of 10×HBSS and 10 mL of water, 70% Percoll was prepared. The 70% Percoll was stored at 4°C until use.

By mixing 7.5 mL of Percoll, 5 mL of 10×HBSS and 37.5 mL of water, 15% Percoll was prepared. The 15% Percoll was stored at 4°C until use.

1-3. Isolation of CT Cells

CT cells were isolated according to the following procedures.

(1) Villous tissue of a human placenta of 20 to 40 weeks of gestation was minced into small pieces.
(2) The minced villous tissue was washed several times with physiological saline.
(3) After adding 50 mL of Accumax and 50 mL of TrypLE, the mixture was incubated at 37°C for 0 minute.
(4) The digested tissue was passed through a 70 um cell strainer.
(5) To the obtained fluid, 20 mL of PBS-DMEM was added.
(6) The mixture was centrifuged at 1,400 rpm for five minutes.
(7) To the cell pellet, 5 mL of PBS-DMEM was added, and the resultant was maintained at 4°C.
(8) (4) to (7) were repeated.
(9) The cell suspension (total volume of 10 mL) was passed through a 70 μm cell strainer.
(10) The cell suspension was centrifuged at 1,400 rpm for five minutes.
(11) To the cell pellet, 1 mL of PBS-DMEM was added.
(12) The cell suspension was laid on a Percoll concentration gradient (15/70%).
(13) The resultant was centrifuged at 1,200 g for 20 minutes.
(14) Cells at the interface of Percoll between 15% and 70% were collected.
(15) To the cells, 20 mL of PBS-EMEM was added.
(16) The mixture was centrifuged at 1,400 rpm for five minutes.
(17) To the cell pellet, 10 mL of PBS was added.
(18) The mixture was centrifuged at 1,400 rpm for five minutes.
(19) To the cell pellet, 1 mL of PBS-EDTA was added to suspend the cells, and the cell suspension was moved to a 14 mL tube.

(20) An anti-CD49f antibody in a volume of 30 μL was added.

(21) While protecting from the light, the cell suspension was incubated at room temperature for 10 minutes.

(22) PE-selection cocktail in a volume of 30 μL was added.

(23) The cell suspension was incubated at room temperature for 10 minutes.

(24) Magnetic particles in a volume of 20 μL were added.

(25) The cell suspension was incubated at room temperature for 10 minutes.

(26) The liquid volume was adjusted to 10 mL by adding PBS-EDTA.

(27) The 14 mL tube was placed in a magnet and left to stand still at room temperature for seven minutes.

(28) The magnet was reversed, and the medium in the tube was drained.

(29) To the 14 mL tube, 10 mL of PBS-EDTA was added.

(30) (27) to (29) were repeated three times.

(31) After centrifuging at 1,400 rpm for five minutes, the CT cells were collected.

2. Production of TS-Like Cells

2-1. Reagents

The reagents used for the test are shown in Table 2.

[Table 2]

| Reagent | Supplier | Cat# |
|---|---|---|
| DMEM/F12 | Wako | 048-29785 |
| 30% BSA | Wako | 017-22231 |
| ITS-X | Wako | 094-06761 |
| L-Ascorbic acid | Wako | 013-12061 |
| VPA | Wako | 227-01071 |
| Penicillin-Streptomycin | Thermo Fisher Scientific | 15140122 |
| PBS | Wako | 166-23555 |
| TrypLE | Thermo Fisher Scientific | 12604-021 |
| EGF | Wako | 053-07871 |
| A83-01 | Wako | 035-24113 |
| CHIR99021 | Wako | 034-23103 |
| Y27632 | Wako | 036-24023 |
| KSR | Thermo Fisher Scientific | 10828028 |
| FBS | 16141-079 | 16141-079 |
| iMatrix511 | Wako | 385-07361 |
| Col IV | Corning | 354233 |
| Doxycycline | Sigma | D9891-1G |
| Cell Banker 1 | Nippon Zenyaku Kogyo | CB011 |
| Nunc 4well plate | Nunc | 176740 |

2-2. Preparation of Media

The reagents used for the media were prepared as follows.

By dissolving 10 mg of A83-01 in 2.37 mL of dimethyl sulfoxide (DMSO), 10 mM A83-01 was prepared. The 10 mM A83-01 was stored at -20°C until use.

By dissolving 5 mg of CHIR99021 in 2.69 mL of DMSO, 4 mM CHIR99021 was prepared. The 4 mM CHIR99021 was stored at -20°C until use.

By dissolving 5 mg of Y27632 in 1.48 mL of sterile water, 10 mM Y27632 was prepared. Y27632 in an amount of 5 mg was stored at -20°C until use.

By dissolving 0.58 g of L-ascorbic acid in 10 mL of water, 200 mM L-ascorbic acid was prepared. The 200 mM L-ascorbic acid was filtered through a filter and then stored at -20°C until use.

By dissolving 500 μg of EGF in 5 mL of PBS/0.2% BSA, 100 μg/mL EGF was prepared. The 100 μg/mL EGF

was stored at -20°C until use.

By dissolving 1 mg of Doxycycline (Dox) in 10 mL of water, 100 μg/mL Dox was prepared. The 100 μg/mL Dox was filtered through a filter and then stored at -20°C until use.

A Basal medium was prepared with the composition shown in Table 3. The Basal medium was stored at 4°C and used within two weeks after the preparation.

| [Table 3] | |
| --- | --- |
| DMEM/F12 | 485 ml |
| BSA | 2.5 ml |
| Penicillin-Streptomycin | 2.5 ml |
| ITS-X | 5 ml |
| KSR | 5 ml |
| 200 mM L-Ascorbic acid | 0.5 ml |

A Term-1 medium was prepared with the composition shown in Table 4. The Term-1 medium was stored at 4°C and used within two weeks after the preparation.

| [Table 4] | |
| --- | --- |
| Basal Medium | 40 ml |
| 10 mM Y27632 | 10 μl |
| 100 μg/ml EGF | 10 μl |
| VPA | 5 μl |
| 10 mM A83-01 | 4 μl |
| 4 mM CHIR99021 | 20 μl |
| FBS | 400 μl |

A Term-2 medium was prepared with the composition shown in Table 5. The Term-2 medium was stored at 4°C and used within two weeks after the preparation.

| [Table 5] | |
| --- | --- |
| Basal Medium | 40 ml |
| 10 mM Y27632 | 10 μl |
| 100 μg/ml EGF | 10 μl |
| VPA | 5 μl |
| 10 mM A83-01 | 4 μl |
| 4 mM CHIR99021 | 20 μl |
| FBS | 200 μl |

2-3. Construction of Dox-Inducible Gene Introduction Lentiviral Vectors

According to the method described by Takahashi et *al.* (Takahashi et al., Proc Natl Acad Sci USA. 2019 Dec 2;116(52):26606-26613.), Dox-inducible gene introduction lentivectors were constructed. The specific method is shown below.

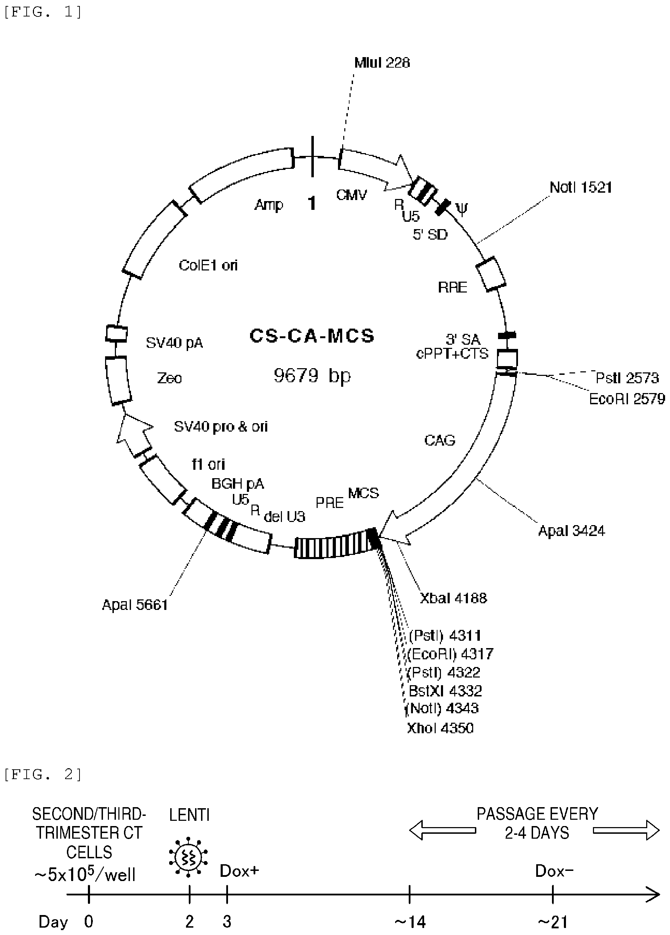

(1) The Tet-On 3G transactivator of pTetOne vector (Takara) was amplified by PCR and cloned into the multicloning site of CS-CA-MCS plasmid (Fig. 1; transferred from Riken BioResource Center (Ibaraki, Japan)) using InFusion HD Cloning kit (Takara) to produce pCS-CA-Tet3G vector.
(2) The CAG promoter of CS-CA-MCS plasmid was replaced with the TRE3Gs promoter of pTetOne vector, and thus pCS-3G vector was produced.
(3) The cDNA of a human gene shown in Table 6 was inserted into the multicloning site of pCS-3G vector, and the vector was used as a Dox-inducible gene introduction lentiviral vector. Dox-inducible gene lentiviral vectors of the 32 kinds of gene shown in Table 6 were each produced.

[Table 6]

| For Reprogramming | | | | | |
|---|---|---|---|---|---|
| BRDT | FOS | HMGA2 | PPARG | TEAD4 | ZFAT |
| DPPA4 | FOXI3 | LIN28A | RNF19B | TFAP2C | ZFP42 |
| E2F5 | FOXO4 | MSX2 | SALL4 | TFCP2L1 | ZNF750 |
| ELF5 | GATA3 | NFE2L3 | SOHLH2 | TP63 | |
| ETS2 | HAND 1 | NR6A1 | SP6 | VGLL1 | |
| For Cell Growth Promotion | | | | | |
| MYC | p53DN | SV40 T | TERT | | |

As the cDNA of p53 dominant negative (p53DN), cDNA amplified from T7-p53-pcDNA3 (addgene) by PCR was used. The p53DN cDNA contained in T7-p53-pcDNA3 encodes a truncated protein (the amino acid sequence from the 300th to 393rd positions) of p53 (AB082923).

2-4. Production of TS-Like Cells

The outline of the time schedule of the operation is shown in Fig. 2. TS-like cells were produced according to the following procedures.

(1) To each well, 1 mL of PBS containing 5 $\mu$L of Col IV and 1 $\mu$L of iMatrix511 was put and left to stand still at 37°C for an hour to coat each well.
(2) PBS/Col IV/iMatrix511 was removed by suction.
(3) The wells were washed with PBS.
(4) PBS was removed by suction.
(5) The Term-1 medium was added to the wells, and the wells were maintained at 37°C for 15 minutes or longer.
(6) In the Term-1 medium, ~$5\times10^5$ CT cells (those isolated in "1. Isolation of CT Cells from Placenta of or after Second Trimester of Pregnancy") were suspended (Day 0) and cultured at 37°C.
(7) On the second day of culture (Day 2), the Term-1 medium was removed by suction, and 2 mL of the Term-2 medium which was warmed to 37°C in advance was added.
(8) Lentivirus particles (5 $\mu$L of pCS-CA-Tet3G vector and 3 to 5 $\mu$L of a Dox-inducible gene introduction lentiviral vector) were added.
(9) On the third day of culture (Day 3), 2 $\mu$L of 100 ug/mL Dox was added.
(10) The medium was changed every three days, and the culture was continued in a Dox-containing Term-2 medium.
(11) On the 21st day of culture (Day 21), the medium was replaced with a Dox-free Term-2 medium, and the culture was continued.

3. Examination of Necessary Genes for TS-Like Cell Production

By introducing into CT cells derived from a placenta of or after the second trimester of pregnancy (second/third-trimester CT cells), genes capable of establishing TS-like cells were examined. The results of examination of the genes listed in Table 6 are shown in Table 7. In Table 7, "Yes" means that TS-like cells could be established, and "No" means that TS-like cells could not be established.

[Table 7]

| Introduced Genes | Establishment of TS-Like Cells (With Dox*) | Establishment of TS-Like Cells (Without Dox**) |
|---|---|---|
| 28 Genes for Reprogramming + p53DN | Yes | Yes |
| 27 Genes for Reprogramming (-SALL4) + p53DN | No | No |
| SALL4 + p53DN | Yes | Yes |

(continued)

| Introduced Genes | Establishment of TS-Like Cells (With Dox*) | Establishment of TS-Like Cells (Without Dox**) |
|---|---|---|
| SALL4 + MYC | Yes | No |
| SALL4 + TERT | No | No |
| SALL4 + SV40 T | No | No |
| * Cultured with continuous existence of Dox. ** Dox was removed on the 21st day. | | |

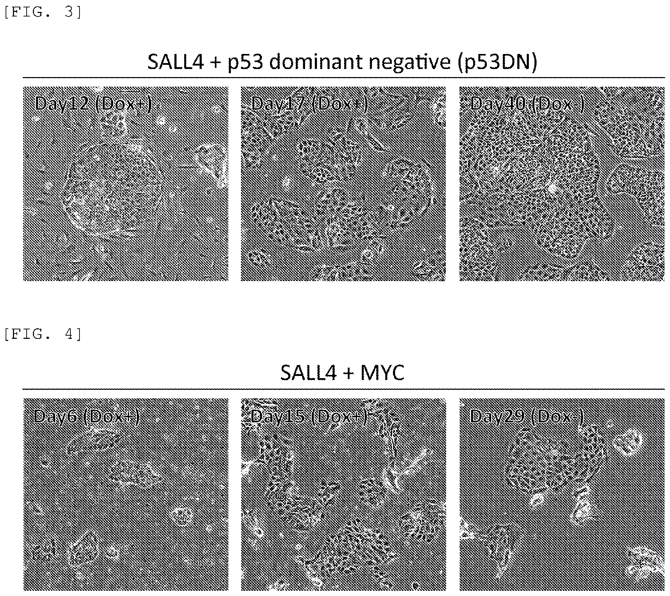

From the results in Table 7, SALL4+p53DN and SALL4+MYC were found to be the combinations of genes capable of establishing TS-like cells in the case of continuous culture in the presence of Dox. Of these, when SALL4+p53DN were used, the TS-like cells were maintained even when the cells were cultured in the absence of Dox from the 21st day of culture (see Fig. 3). On the other hand, when SALL4+MYC were used, the TS-like cells could not be maintained in the absence of Dox (see Fig. 4: the case of SALL4+MYC is shown). The results show that TS-like cells induced from second/third-trimester CT cells with the combination of SALL4+MYC require continuous expression of SALL4+MYC for the maintenance. On the other hand, the results show that TS cells induced with the combination of SALL4+p53DN do not require continuous expression of SALL4+p53DN for the maintenance.

4. Gene Expression Analysis of TS-Like Cells

4-1. Reagents and Devices

The reagents and the devices used for the test are shown below.

RNeasy mini kit (Qiagen, Cat#: 74104)
TruSeq RNA Sample Prep Kit (Illumina, Cat#: RS-122-2001)
Next generation sequencer (HiSeq2500 of Illumina was used.)
Next generation sequencer analysis software Strand NGS (Digital Biology)

4-2. Gene Expression Analysis

Gene expression analysis was conducted, and the similarities of TS-like cells induced from second/third-trimester CT cells (second/third-trimester TS-like cells), TS-like cells induced from first-trimester placenta-derived CT cells (first-trimester TS-like cells), TS cells, second/third-trimester CT cells and first-trimester CT cells were examined.
As the second/third-trimester TS-like cells, those produced by introducing SALL4+p53DN into second/third-trimester CT cells were used. For the analysis, cells which were cultured in a Dox-free medium on and after the 21st day of culture were used (see Fig. 2).
The first-trimester TS-like cells were produced according to the method described in patent No. 6400832.
Moreover, gene expression analysis was conducted also regarding cells obtained by treating the second/third-trimester CT cells by a method similar to the method described in patent No. 6400832 (the method for inducing first-trimester TS-like cells).
According to the following method, gene expression analysis was conducted.

(1) Total RNA was extracted from the cells using RNeasy mini kit.
(2) A library for a next generation sequencer was produced using TruSeq RNA Sample Prep Kit.
(3) The sequence was analyzed using a next generation sequencer.
(4) The expression levels of genes (TPM values: transcripts per million) were calculated using next generation sequencer analysis software, Strand NGS.

4-3. Main Component Analysis

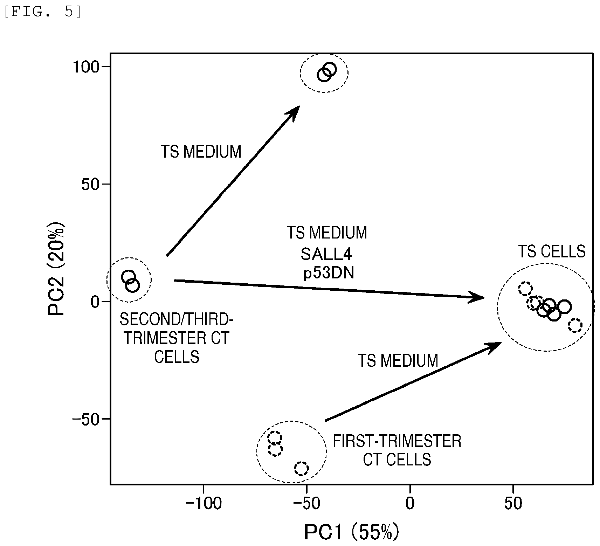

Based on the gene expression levels analyzed with the next generation sequencer, main component analysis was conducted using R. The results are shown in Fig. 5.
As shown in Fig. 5, it was observed that the second/third-trimester TS-like cells show a similar expression profile to those of the TS cells and the first-trimester TS-like cells. On the other hand, when the second/third-trimester CT cells were induced by a similar method to that of the first-trimester CT cells, an expression profile which was different from that of the TS cells was shown.

4-4. Expression Analysis of TS Cell Markers

Regarding the first-trimester TS-like cells and the second/third-trimester TS-like cells, the expression of ELF5, ZNF750 and CDX2 was observed. ELF5 and ZNF750 are positive markers of TS cells, and CDX2 is a negative marker of TS cells.

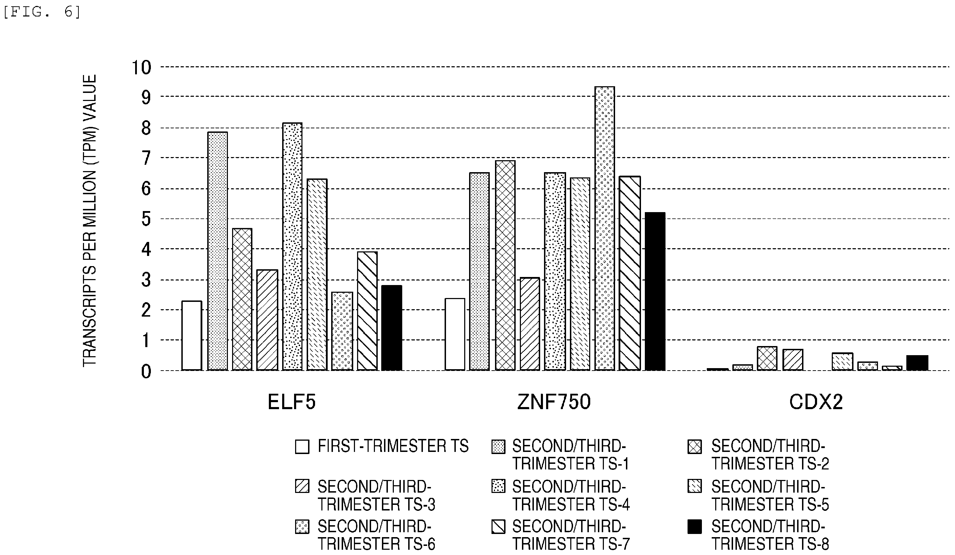

The results are shown in Fig. 6. One clone of first-trimester TS-like cells (first-trimester TS) was analyzed, and eight clones of second/third-trimester TS-like cells (second/third-trimester TS-1 to 8) were analyzed. All the clones of first-trimester TS-like cells and second/third-trimester TS-like cells were ELF5 and ZNF750 positive and CDX2 negative. The results show that the second/third-trimester TS-like cells are similar to the TS cells.

5. Induced Differentiation into EVTs or STs

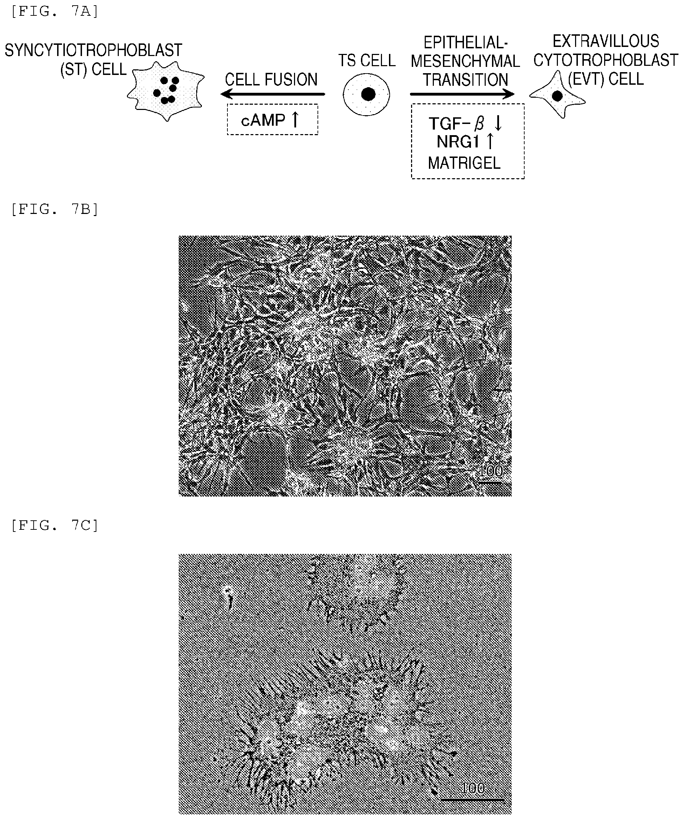

It was confirmed that second/third-trimester TS-like cells have potential to differentiate into placenta constituting cells. TS cells undergo epithelial-mesenchymal transition in an environment with a low TGF-β concentration and high NRG1 and Matrigel concentrations, and the differentiation thereof into extravillous cytotrophoblast (EVT) cells is induced (see Fig. 7A). Moreover, TS cells are cell-induced in an environment with a high cAMP concentration, and the differentiation thereof into syncytiotrophoblast (ST) cells is induced (see Fig. 7A). It was observed whether differentiation from second/third-trimester TS-like cells into EVTs and STs would also be induced, like TS cells, by culturing the cells in the environments.

As the second/third-trimester TS-like cells for induced differentiation, second/third-trimester TS-like cells produced by introducing SALL4+p53DN into second/third-trimester CT cells were used. Moreover, cells which were cultured in a Dox-free medium on and after the 21st day of culture were used (see Fig. 2).

5-1. Induced Differentiation into EVTs

5-1-1. Preparation of EVT Medium

By adding the following components to DMEM/F12 (048-29785; Fujifilm Wako, Osaka, Japan), an EVT medium was prepared.

> 0.5% Penicillin-Streptomycin (Thermo Fisher Scientific)
> 0.3% BSA (Fujifilm Wako)
> 1% ITS-X supplement (Fujifilm Wako)
> 100 ng/ml NRG1 (5218SC; Cell Signaling)
> 7.5 μM A83-01 (Fujifilm Wako)
> 2.5 μM Y27632 (Fujifilm Wako)
> 4% KSR (Thermo Fisher Scientific)

5-1-2. Induced Differentiation into EVTs

According to the following procedures, differentiation from the second/third-trimester TS-like cells into EVTs was induced.

(1) The second/third-trimester TS-like cells were inoculated on a 1 μg/mL Col IV-coated plate (Corning) containing the EVT medium at a density of 8,000 cells/cm². 
(2) Matrigel (Corning) was added at 2% of the medium volume.
(3) On the third day after the inoculation, the medium was replaced with the EVT medium which did not contain NRG1 (Cell Signaling), and Matrigel (Corning) was added at 0.5% of the medium volume.
(4) On the sixth day after the inoculation, the medium was replaced with the EVT medium which did not contain NRG1 (Cell Signaling) or KSR (Thermo Fisher Scientific), and Matrigel (Corning) was added at 0.5% of the medium volume.
(5) On the sixth to eighth day after replacing the medium, the cells were observed under a microscope.

5-1-3. Results

A microscope image of the cells after the induced differentiation is shown in Fig. 7B. As a result of the observation under a microscope, it was confirmed that the differentiation of the second/third-trimester TS-like cells into EVTs was induced.

5-2. Induced Differentiation into STs

5-2-1. Preparation of ST Medium

By adding the following components to DMEM/F12 (048-29785; Fujifilm Wako, Osaka, Japan), an ST medium was prepared.

> 0.1 mM 2-mercaptoethanol (Thermo Fisher Scientific)
> 0.5% Penicillin-Streptomycin (Thermo Fisher Scientific)
> 0.3% BSA (Fujifilm Wako)
> 1% ITS-X supplement (Fujifilm Wako)
> 2.5 $\mu$M Y27632 (Fujifilm Wako)
> 2 $\mu$M forskolin (Fujifilm Wako)
> 4% KSR (Thermo Fisher Scientific)

5-2-2. Induced Differentiation into STs

According to the following procedures, differentiation from the second/third-trimester TS-like cells into STs was induced.

> (1) The second/third-trimester TS-like cells were inoculated on a 2.5 $\mu$g/mL Col IV-coated plate (Corning) containing the ST medium at a density of 10,000 cells/cm$^2$.
> (2) On the third day after the inoculation, the ST medium was changed.
> (3) On the sixth day after the inoculation, the cells were observed under a microscope.

5-2-3. Results

A microscope image of the cells after the induced differentiation is shown in Fig. 7C. As a result of the observation under a microscope, it was confirmed that the differentiation of the second/third-trimester TS-like cells into STs was induced.

6. Marker Distinguishing First-Trimester CT Cells and Second/Third-Trimester CT Cells

Regarding the first-trimester CT cells and the second/third-trimester CT cells, gene expression analysis was conducted by a similar method to that in "4-2. Gene Expression Analysis" above, and a marker which distinguishes the first-trimester CT cells and the second/third-trimester CT cells was examined.

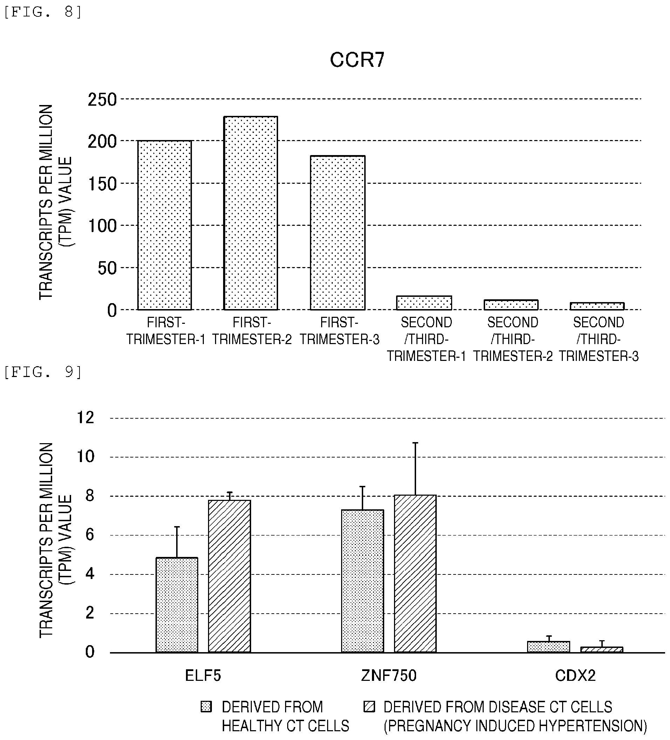

As a result, CCR7 was found as a gene which is expressed in the first-trimester CT cells but which is not expressed in the second/third-trimester CT cells. In Fig. 8, the results of expression analysis of CCR7 in three clones of first-trimester CT cells (first-trimester-1, first-trimester-2 and first-trimester-3) and three clones of second/third-trimester CT cells (second/third-trimester-1, second/third-trimester-2 and second/third-trimester-3) are shown. It was confirmed that the first-trimester CT cells were CCR7 positive and that the second/third-trimester CT cells were CCR7 negative.

7. Isolation of CT Cells from Placenta of Pregnancy Induced Hypertension Patient

7-1. Reagents and Instruments

The reagents and the instruments used for the test are as shown in Table 1 above.

7-2. Preparation of Medium

The reagents and the medium were prepared as described in "1-2. Preparation of Medium".

7-3. Isolation of CT Cells

Villous tissue of the human placenta of a pregnancy induced hypertension patient in the 20 to 40 weeks of gestation was minced into small pieces. The subsequent isolation step was conducted in a similar manner to that of (2) to (31) in "1-3. Isolation of CT Cells". The obtained CT cells are also referred to as "disease CT cells" below. CT cells were also isolated from a healthy human placenta (also referred to as "healthy CT cells" below) in a similar manner.

8. Production of TS-Like Cells from Disease CT Cells 8-1. Reagents

The reagents used for the test are as shown in Table 2 above.

8-2. Preparation of Media

The reagents used for the media were prepared as described in "2-2. Preparation of Media". A Basal medium was prepared as shown in Table 3 above, stored at 4°C and used within two weeks after the preparation. A Term-1 medium was prepared as shown in Table 4 above, stored at 4°C and used within two weeks after the preparation. A Term-2 medium was prepared as shown in Table 5 above, stored at 4°C and used within two weeks after the preparation.

8-3. Construction of Dox-Inducible Gene Introduction Lentiviral Vectors

As described in "2-3. Construction of Dox-Inducible Gene Introduction Lentiviral Vectors", Dox-inducible gene introduction lentivectors were constructed. Regarding the genes inserted into the multicloning site of pCS-3G vector, SALL4 was selected as a gene for reprogramming, and p53DN was selected for cell growth.

8-4. Production of TS-Like Cells from Disease CT Cells (Disease CT Cell-Derived TS Cells)

TS-like cells were produced in a similar manner to that in "2-4. Production of TS-Like Cells" except that the disease CT cells were used. TS-like cells were produced in a similar manner also from the healthy CT cells.

9. Gene Expression Analysis of Disease CT Cell-Derived TS-Like Cells

9-1. Reagents and Devices

The reagents and the devices used for the test are as described in "4-1. Reagents and Devices".

9-2. Gene Expression Analysis

According to the procedures described in "4-2. Gene Expression Analysis", gene expression analysis was conducted.

9-3. Expression Analysis of TS Cell Markers

Regarding the disease CT cell-derived TS-like cells, the expression of ELF5, ZNF750 and CDX2 was observed. Moreover, regarding the healthy CT cell-derived TS-like cells, the expression of ELF5, ZNF750 and CDX2 was observed in a similar manner.
The results are shown in Fig. 9. Three clones of disease CT cell-derived TS-like cells and four clones of healthy CT cell-derived TS-like cells were analyzed. All the clones were ELF5 and ZNF750 positive and CDX2 negative. The results show that the disease CT cell-derived TS-like cells are similar to the TS cells.

Industrial Applicability

According to the invention, a method for producing TS-like cells from trophoblast cells derived from a placenta of or after the second trimester of pregnancy and TS-like cells produced by the production method are provided. The TS-like cells provided by the invention can be used for research on early development of placental mammals, analytical research of placental functions and research on the pathological conditions of pregnancy-associated diseases or development of therapeutic methods thereof. Moreover, application to regenerative medicine is also possible.

**Claims**

1. A trophoblast stem cell-like cell having potential to differentiate into a placenta-constituting cell

   which is induced from a trophoblast cell derived from a placenta of or after the second trimester of pregnancy and which comprises a SALL4 gene functionally linked to a first exogenous inducible promoter.

2. The trophoblast stem cell-like cell according to claim 1,

   wherein the SALL4 gene is
   an exogenous SALL4 gene functionally linked to the first exogenous inducible promoter or
   an endogenous SALL4 gene functionally linked to the first exogenous inducible promoter.

3. The trophoblast stem cell-like cell according to claim 1 or 2 which has at least one feature selected from the group consisting of (a) and (b) below:

   (a) the activity of p53 is suppressed compared to that in the trophoblast cell; and
   (b) the expression of a MYC gene is promoted compared to that in the trophoblast cell.

4. The trophoblast stem cell-like cell according to any one of claims 1 to 3 which further comprises an exogenous gene functionally linked to a second exogenous inducible promoter,
   wherein the exogenous gene is at least one selected from the group consisting of an exogenous p53 dominant negative gene and an exogenous MYC gene.

5. The trophoblast stem cell-like cell according to claim 4, wherein the exogenous gene is the exogenous p53 dominant negative gene.

6. A method for producing a trophoblast stem cell-like cell having potential to differentiate into a placenta-constituting cell, comprising

   (i) a step of preparing a trophoblast cell derived from a placenta of or after the second trimester of pregnancy,
   (ii) a step of inducing the expression of a SALL4 gene in the trophoblast cell and
   (iii) a step of conducting at least one selected from the group consisting of (A) and (B) below:

      (A) suppressing the activity of p53 of the trophoblast cell; and
      (B) inducing the expression of a MYC gene in the trophoblast cell.

7. The method for producing a trophoblast stem cell-like cell according to claim 6,

   wherein the step in (ii) comprises
   introducing a polynucleotide containing an exogenous SALL4 gene functionally linked to a first exogenous inducible promoter into the trophoblast cell or
   introducing a polynucleotide containing the first exogenous inducible promoter into the trophoblast cell at the upstream of an endogenous SALL4 gene in such a manner that the endogenous SALL4 gene is functionally linked to the first exogenous inducible promoter and
   inducing the expression of the exogenous SALL4 gene or the endogenous SALL4 gene with a first inducing factor which induces the transcriptional activity of the first exogenous inducible promoter.

8. The method for producing a trophoblast stem cell-like cell according to claim 7,

   wherein the (A) in the step in (iii) comprises
   introducing a polynucleotide containing an exogenous p53 dominant negative gene functionally linked to a second exogenous inducible promoter into the trophoblast cell and
   inducing the expression of the exogenous p53 dominant negative gene with a second inducing factor which induces the transcriptional activity of the second exogenous inducible promoter, and
the (B) in the step in (iii) comprises
introducing an exogenous MYC gene functionally linked to the second exogenous inducible promoter into the trophoblast cell and
inducing the expression of the exogenous MYC gene with a second inducing factor which induces the transcriptional activity of the second exogenous inducible promoter.

9. The method for producing a trophoblast stem cell-like cell according to any one of claims 6 to 8, wherein the step in (iii) comprises the (A).

[FIG. 1]

[FIG. 2]

[FIG. 3]

## SALL4 + p53 dominant negative (p53DN)

[FIG. 4]

## SALL4 + MYC

[FIG. 5]

[FIG. 6]

[FIG. 7A]

[FIG. 7B]

[FIG. 7C]

[FIG. 8]

[FIG. 9]

**INTERNATIONAL SEARCH REPORT**

| | International application No. |
|---|---|
| | PCT/JP2020/027766 |

**A. CLASSIFICATION OF SUBJECT MATTER**
Int.Cl. C12N5/10(2006.01)i, C12N15/10(2006.01)i
FI: C12N15/10, C12N5/10

According to International Patent Classification (IPC) or to both national classification and IPC

**B. FIELDS SEARCHED**

Minimum documentation searched (classification system followed by classification symbols)
Int.Cl. C12N5/10, C12N15/10

Documentation searched other than minimum documentation to the extent that such documents are included in the fields searched
Published examined utility model applications of Japan    1922-1996
Published unexamined utility model applications of Japan  1971-2020
Registered utility model specifications of Japan          1996-2020
Published registered utility model applications of Japan  1994-2020

Electronic data base consulted during the international search (name of data base and, where practicable, search terms used)
JSTPlus/JMEDPlus/JST7580(JDreamIII),
CAplus/MEDLINE/EMBASE/BIOSIS/WPIDS(STN), AGRICOLA(STN), FSTA(STN),
TOXCENTER(STN)

**C. DOCUMENTS CONSIDERED TO BE RELEVANT**

| Category* | Citation of document, with indication, where appropriate, of the relevant passages | Relevant to claim No. |
|---|---|---|
| X<br>A | 岡江寛明，ヒト疾患未分化胎盤幹細胞モデルの作製と病態解明，上原記念生命科学財団研究報告集 [online], 04 February 2020, vol. 33, Internet: <https://www.ueharazaidan.or.jp/houkokushu/Vol.33/pdf/report/137_report.pdf>, [retrieved on 07 September 2020], entire text, all drawings, (OKAE, Hiroaki), non-official translation (Creation of undifferentiated placental stem cell model of human disease and elucidation of pathophysiology, The Uehara Memorial Foundation Research Report Collection) | 1-4, 6, 7<br>5, 8, 9 |
| A | OKAE, H. et al., Derivation of human trophoblast stem cells, Cell Stem Cell, 04 January 2018, vol. 22, pp. 50-63, entire text, all drawings | 1-9 |

☒ Further documents are listed in the continuation of Box C.   ☐ See patent family annex.

| * Special categories of cited documents: | |
|---|---|
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 09 September 2020 | 24 September 2020 |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.

PCT/JP2020/027766

C (Continuation).   DOCUMENTS CONSIDERED TO BE RELEVANT

| Category* | Citation of document, with indication, where appropriate, of the relevant passages | Relevant to claim No. |
|---|---|---|
| A | 小林記緒, 他, ヒト胚性幹細胞の栄養膜幹細胞への分化転換, 第42回日本分子生物学会年会プログラム・要旨集, 19 November 2019, [1P-0326], entire text, (KOBAYASHI, Kio et al.), non-official translation (Transdifferentiation of human embryonic stem cells to trophoblast stem cells, Program / Abstracts of the 42nd Annual Meeting of the Molecular Biology Society of Japan) | 1-9 |
| A | 岡江寛明, 他, なぜ絨毛癌は稀なのか？ ヒト栄養膜幹細胞を用いた胎盤の腫瘍化抑制機構の解析, 第42回日本分子生物学会年会プログラム・要旨集, 19 November 2019, [3AW-18-6], entire text, (OKAE, Hiroaki et al.), non-official translation (Why is choriocarcinoma rare? Analysis of placental tumorigenesis suppression mechanism using human trophoblast stem cells, Program / Abstracts of the 42nd Annual Meeting of the Molecular Biology Society of Japan) | 1-9 |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 6400832 A

**Non-patent literature cited in the description**

- **OKAE H et al.** Derivation of Human Trophoblast Stem Cells. *Cell Stem Cell,* January 2018, vol. 22, 50-63

- **SAMBROOK et al.** Molecular Cloning-A LABORATORY MANUAL THIRD EDITION. Cold Spring Harbor Laboratory Press
- **TAKAHASHI et al.** *Proc Natl Acad Sci USA.,* 02 December 2019, vol. 116 (52), 26606-26613